# EUROPEAN PATENT APPLICATION

(11) **EP 3 213 739 A1**
(43) Date of publication of application: **06.09.2017**
(21) Application number: 15854398.3
(22) Date of filing: 20.10.2015
(51) Int. Cl.: A61K 8/33, A61K 8/365, A61K 8/37, A61K 31/22, A61P 17/00, A61P 43/00, A61Q 19/08

(54) **AGENT FOR IMPROVING MITOCHONDRIAL FUNCTION AND ANTI-AGING COMPOSITION**

(30) Priority: 29.10.2014 JP 2014220578
(71) Applicant: Showa Denko K.K., Tokyo 105-8518 (JP)
(72) Inventor: SAEKI Yuko, Tokyo 105-8518 (JP)
(74) Representative: Strehl Schübel-Hopf & Partner
(86) International application number: PCT/JP2015/079543
(87) International publication number: WO 2016/067968

(57) **Abstract**

Provided is an agent for improving mitochondrial function including a hydroxycitric acid derivative represented by the following Formula (I) or a salt thereof, as an active ingredient [(In Formula (I), R¹ and R² each independently represent a hydrogen atom or any one group selected from the group represented by the following Formula (Ia) (here, R¹ and R² do not represent hydrogen atoms at the same time), X¹ to X³ each independently represent a nitrogen or an oxygen atom, and R³, R⁴, R⁵, R^{3'}, R^{4'}, and R^{5'} each independently represent a hydrogen atom, or a chained hydrocarbon group (here, in a case where X¹, X², and X³ each independently represent an oxygen atom, corresponding R^{3'}, R^{4'}, or R^{5'} does not exist). In Formula (Ia), R⁶ to R⁸ each independently represent a hydrogen atom, an aryl group, or a chained hydrocarbon group having 1 to 30 carbon atoms].

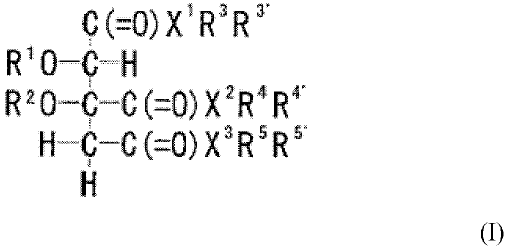

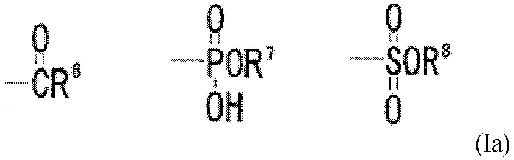

## Description

### Technical Field

The present invention relates to an agent for improving mitochondrial function and an anti-aging composition. Priority is claimed on Japanese Patent Application No. 2014-220578, filed on October 29, 2014, the content of which is incorporated herein by reference.

### Background Art

Mitochondria are cell organelles responsible for the generation of energy in cells and consume oxygen during an oxidative phosphorylation reaction to generate ATP via an electron transport chain. Protons generated during this reaction process are pumped out to the space between the outer membrane and the inner membrane of the mitochondria (intermembrane space), and therefore a potential difference (mitochondrial membrane potential) is generated. Next, an ATP synthase harnesses the protons in the intermembrane space to produce ATP. In addition, stress such as DNA damage due to ultraviolet radiation changes the mitochondrial membrane potential via p53- or BCL-family proteins and induces cell death, called apoptosis. It has been reported that in aged cells or cells damaged by ultraviolet rays, active oxygen or a lipid peroxidation product resulting therefrom is generated (NPLs 1 and 2), which leads to the decrease in the energy generation of the mitochondria and the increase of apoptosis (NPL 3). Accordingly, it is considered that mitochondria are closely related to aging and photoaging.

It is considered that by improving mitochondrial function, it is possible to activate aged cells or cells damaged by stress such as ultraviolet rays. Sphingomyelin as an agent for improving mitochondrial function and glutathione as an antioxidative substance have been reported so far (PTL 1 and NPL 4), but the effect thereof is not yet sufficient.

### Citation List

### Patent Literature

[PTL 1] Japanese Unexamined Patent Application, First Publication No. 2011-157328

### Non-Patent Literature

[NPL 1] Journal of Dermatological Science 14 (1997) 207-216
[NPL 2] Free Radic Res. 2001 Jun; 34(6):583-94.
[NPL 3] Ageing Research Reviews, 5 (2006) 179-195
[NPL 4] A Toxicology in Vitro 18 (2004) 71-77

### Summary of the Invention

### Problems to be Solved

An object of the present invention is to provide an agent for improving mitochondrial function capable of improving mitochondrial function, and an anti-aging composition exhibiting effect on preventing skin aging and improving aging symptoms.

### Means for Solving the Problems

In order to achieve the above object, the present invention provides means as follows.
[1] An agent for improving mitochondrial function including a hydroxycitric acid derivative represented by the following Formula (I) or a salt thereof, as an active ingredient: 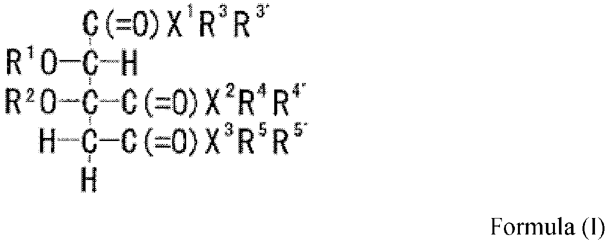
   (In Formula (I), R¹ and R² each independently represent a hydrogen atom or any one group selected from the group represented by the following Formula (Ia) (here, R¹ and R² do not represent hydrogen atoms at the same time), X¹ to X³ each independently represent a nitrogen or an oxygen atom, and R³, R⁴, R⁵, R^{3'}, R^{4'}, and R^{5'} each independently represent a hydrogen atom, or a chained hydrocarbon group having 1 to 30 carbon atoms which may have a branch or an unsaturated bond (here, in a case where X¹, X², and X³ each independently represent an oxygen atom, corresponding R^{3'}, R^{4'}, or R^{5'} does not exist) 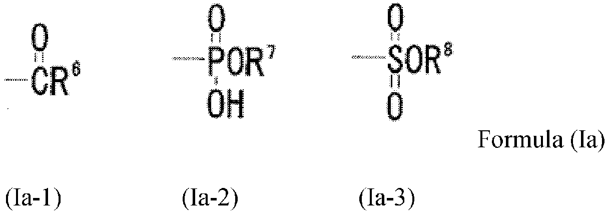
   (In Formula (Ia), R⁶ to R⁸ each independently represent a hydrogen atom, an aryl group, or a chained hydrocarbon group having 1 to 30 carbon atoms which may have a branch, an unsaturated bond, or a substituent).
[2] The agent for improving mitochondrial function according to [1], in which in Formula (Ia), R⁶ represents a chained hydrocarbon group having 7 to 23 carbon atoms which may have a branch, an unsaturated bond, or a substituent, and R⁷ and R⁸ each independently represent a hydrogen atom, or a chained hydrocarbon group having 8 to 24 carbon atoms which may have a branch, an unsaturated bond, or a substituent.
[3] The agent for improving mitochondrial function according to [1], in which in Formula (I), X¹ to X³ all represent oxygen atoms, and R³ to R⁵ each independently represent a hydrogen atom, or a chained hydrocarbon group having 1 to 30 carbon atoms which may have a branch or an unsaturated bond, and in Formula (Ia), R⁶ represents a chained hydrocarbon group having 7 to 23 carbon atoms which may have a branch, an unsaturated bond, or a substituent, and R⁷ and R⁸ each independently represent a hydrogen atom, or a chained hydrocarbon group having 8 to 24 carbon atoms which may have a branch, an unsaturated bond, or a substituent.
[4] The agent for improving mitochondrial function according to [1], in which in Formula (I), R² represents a hydrogen atom, X¹ to X³ all represent oxygen atoms, and R³ to R⁵ all represent hydrogen atoms, and in Formula (Ia), R⁶ represents a chained hydrocarbon group having 13 to 21 carbon atoms which may have a branch, an unsaturated bond, or a substituent.
[5] The agent for improving mitochondrial function according to [1], in which in Formula (I), R² represents a hydrogen atom, X¹ to X³ all represent oxygen atoms, and R³ to R⁵ each independently represent a hydrogen atom, or a chained hydrocarbon group having 1 to 30 carbon atoms which may have a branch or an unsaturated bond (here, R³ to R⁵ do not represent hydrogen atoms at the same time), and in Formula (Ia), R⁶ represents a chained hydrocarbon group having 13 to 21 carbon atoms which may have a branch, an unsaturated bond, or a substituent.
[6] The agent for improving mitochondrial function according to [1], in which Formula (I) represents a hydroxycitric acid derivative represented by the following Formula (IIa) or a salt thereof: 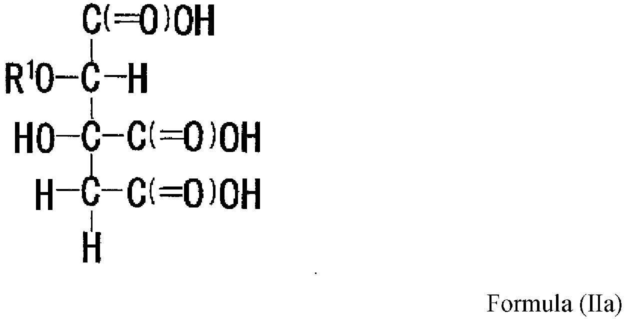 (in Formula (IIa), R¹ represents a group represented by the following Formula (Ia-1)), 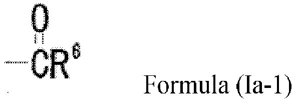 (in Formula (Ia-1), R⁶ represents a chained hydrocarbon group having 7 to 23 carbon atoms which may have a branch, an unsaturated bond, or a substituent).
[7] A method for producing the agent for improving mitochondrial function according to any one of [1] to [6], the method including producing the hydroxycitric acid derivative or a salt thereof by reacting hydroxycitric acid and/or an alkali metal salt thereof and/or an alkaline earth metal salt thereof with a carboxylic acid derivative, a phosphoric acid derivative or a sulfonic acid derivative in a solvent.
[8] A method for producing the agent for improving mitochondrial function according to any one of [1] to [6], the method including producing the hydroxycitric acid derivative or a salt thereof by the following steps:
   a first step of producing a hydroxycitric acid (tri)ester by reacting hydroxycitric acid and/or an alkali metal salt thereof and/or an alkaline earth metal salt thereof with alcohol in a solvent,
   a second step of esterifying a hydroxyl group of a compound obtained in the first step by reacting an aliphatic carboxylic acid derivative and the compound obtained in the first step, and
   a third step of cleaving a part or all of ester bond moieties formed in the first step from ester bond moieties of the compound obtained in the second step.
[9] An anti-aging composition including a hydroxycitric acid derivative represented by the following Formula (I) or a salt thereof, as an active ingredient: 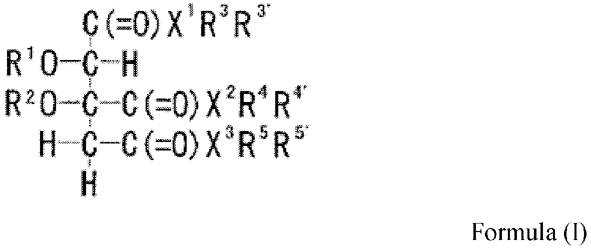
(in Formula (I), R¹ and R² each independently represent a hydrogen atom or any one group selected from the group represented by the following Formula (Ia) (here, R¹ and R² do not represent hydrogen atoms at the same time), X¹ to X³ each independently represent a nitrogen or an oxygen atom, and R⁴, R⁵, R^{3'}, R^{4'}, and R^{5'} each independently represent a hydrogen atom, or a chained hydrocarbon group having 1 to 30 carbon atoms which may have a branch or an unsaturated bond (here, in a case where X¹, X², and X³ each independently represent an oxygen atom, corresponding R^{3'}, R^{4'}, or R^{5'} does not exist), 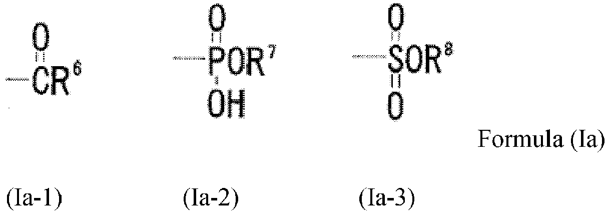 (in Formula (Ia), R⁶ to R⁸ each independently represent a hydrogen atom, an aryl group, or a chained hydrocarbon group having 1 to 30 carbon atoms which may have a branch, an unsaturated bond, or a substituent).

### Effects of the Invention

According to an agent for improving mitochondrial function of the present invention, it is possible to improve mitochondrial function.

In addition, an anti-aging composition of the present invention is effective on preventing or improving aging of cells and skin tissues, such as the formation of wrinkles, sagging, or the like.

### Embodiments for Carrying Out the Invention

### «Agent for Improving Mitochondrial Function»

### <Hydroxycitric Acid Derivative or Salt Thereof>

An agent for improving mitochondrial function of the present invention comprises a hydroxycitric acid derivative represented by the following Formula (I) or a salt thereof, as an active ingredient:

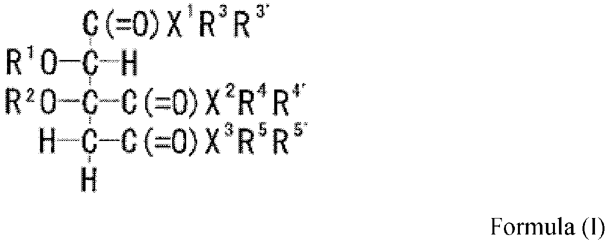

(In Formula (I), R¹ and R² each independently represent a hydrogen atom or any one group selected from the group represented by the following Formula (Ia) (here, R¹ and R² do not represent hydrogen atoms at the same time), X¹ to X³ each independently represent a nitrogen or an oxygen atom, and R⁴, R⁵, R^{3'}, R^{4'}, and R^{5'} each independently represent a hydrogen atom, or a chained hydrocarbon group having 1 to 30 carbon atoms which may have a branch or an unsaturated bond (here, in a case where X¹, X², and X³ each independently represent an oxygen atom, corresponding R^{3'}, R^{4'}, or R^{5'} does not exist)

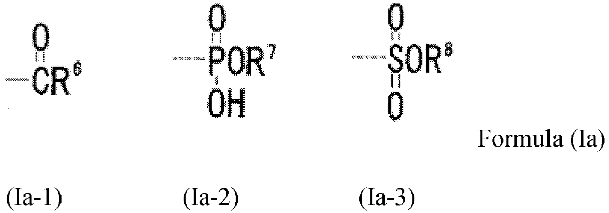

(In Formula (Ia), R⁶ to R⁸ each independently represent a hydrogen atom, an aryl group, or a chained hydrocarbon group having 1 to 30 carbon atoms which may have a branch, an unsaturated bond, or a substituent).

The hydroxycitric acid derivative according to the agent for improving mitochondrial function of the present invention is a compound represented by the following Formula (I):

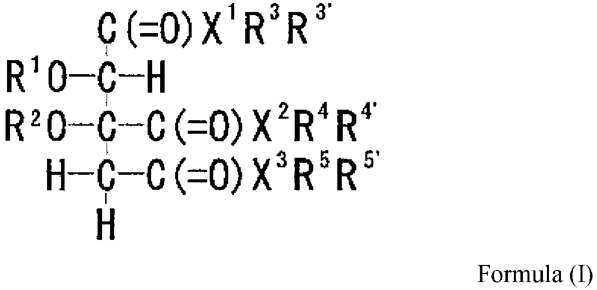

In Formula (I), R¹ and R² each independently represent a hydrogen atom or any one group selected from the group represented by the following Formula (Ia) (here, R¹ and R² do not represent hydrogen atoms at the same time).

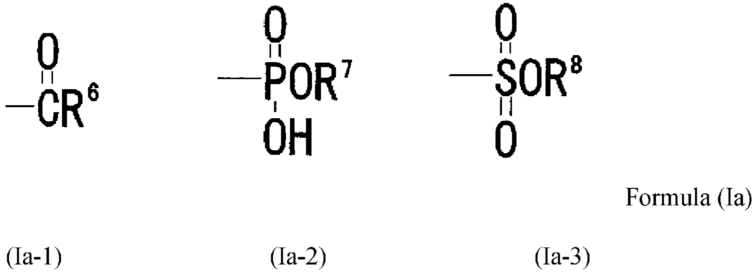

Specifically, it is preferable that at least one of R¹ and R² represent any one group selected from the group represented by Formula (Ia), and one of R¹ and R² may represent the above group, or both of R¹ and R² may represent the above group. Among these, an embodiment in which one of R¹ and R² represents the above group is preferable, and an embodiment in which R¹ is any one group selected from the group represented by Formula (Ia), and R² is a hydrogen atom is more preferable.

In Formula (Ia), R⁶ to R⁸ each independently represent a hydrogen atom, an aryl group, or a chained hydrocarbon group having 1 to 30 carbon atoms which may have a branch, an unsaturated bond, or a substituent.

Examples of the aryl group include a phenyl group, a naphthyl group, a furyl group, a thienyl group, and a pyridyl group. Examples of the chained hydrocarbon group having 1 to 30 carbon atoms which may have a branch, an unsaturated bond, or a substituent include a substance constituting a part of an acyl group which will be described below as an example of R¹ and/or R², and the like.

Among these, it is preferable that R⁶ to R⁸ each independently represent a hydrogen atom, or a chained hydrocarbon group having 1 to 30 carbon atoms which may have a branch, an unsaturated bond, or a substituent.

More specifically, R⁶ represents a chained hydrocarbon group having 7 to 23 carbon atoms, preferably having 13 to 21 carbon atoms, which may have a branch, an unsaturated bond, or a substituent, and more preferably represents a chained saturated hydrocarbon group having 13 to 21 carbon atoms which may have a branch. R⁷ and R⁸ each independently represent a hydrogen atom, or a chained hydrocarbon group having 8 to 24 carbon atoms which may have a branch, an unsaturated bond, or a substituent, preferably represent a hydrogen atom or a chained hydrocarbon group having 14 to 22 carbon atoms, and more preferably a hydrogen atom.

Among these, it is desirable that any one group selected from the group represented by Formula (Ia) be a group having 8 to 24 carbon atoms, preferably 14 to 22 carbon atoms.

Examples of R⁶, R⁷, and R⁸ include groups specifically exemplified as R⁴, R⁵, R^{3'}, R^{4'}, and R^{5'} which will be described below.

Examples of the substituent include a halogen atom, an amino group, a cyano group, an alkoxy group, and a nitro group, and the like.

Specific examples of the derivative of which a hydroxyl group in a molecule is modified include a compound of which R¹ and/or R² in Formula (I) are any one of a hexanoyl group, a 2-methylpentanoyl group, a 3-methylpentanoyl group, a 4-methylpentanoyl group, a 2-ethylbutanoyl group, a heptanoyl group, a 2-methylhexanoyl group, a 3-methylhexanoyl group, a 4-methylhexanoyl group, a 2-ethylpentanoyl group, a 3-ethylpentanoyl group, an octanoyl group, a 2-methylheptanoyl group, a 3-methylheptanoyl group, a 4-methylheptanoyl group, a 5-methylheptanoyl group, a 6-methylheptanoyl group, a 2-ethylhexanoyl group, a 3-ethylhexanoyl group, a 4-ethylhexanoyl group, a 2-propylpentanoyl group, a nonanoyl group, a decanoyl group, a undecanoyl group, a 10-undecenoyl group, a dodecanoyl group, a tridecanoyl group, a tetradecanoyl group, a pentadecanoyl group, a hexadecanoyl group, a 9-hexadecenoyl group, a heptadecanoyl group, an octadecanoyl group, an isostearyl group, a cis-9-octadecenoyl group, a 11-octadecenoyl group, a cis,cis-9,12-octadecadienoyl group, a 9,12,15-octadecatrienoyl group, a 6,9,12-octadecatrienoyl group, a 9,11,13-octadecatrienoyl group, a nonadecanoyl group, a 2,6,10,14-tetramethylbentadecanoyl group, an icosanoyl group, a 8,11-icosadienoyl group, a 5,8,11-icosatrienoyl group, a 5,8,11,14-icosatetraenoyl group, a 3,7,11,15-tetramethylhexadecanoyl group, a henicosanoyl group, and a docosanoyl group.

Among these, preferable examples include a compound of which R¹ and/or R² in Formula (I) are any one of an octanoyl group, a decanoyl group, an undecanoyl group, a dodecanoyl group, a hexadecanoyl group, an octadecanoyl group, and an isostearyl group.

More preferable examples include a compound of which R¹ is any one of an octanoyl group, a decanoyl group, an undecanoyl group, a dodecanoyl group, a hexadecanoyl group, an octadecanoyl group, and an isostearyl group, and R² is a hydrogen atom.

Next, in Formula (I), X¹ to X³ each independently represent a nitrogen or an oxygen atom, and R⁴, R⁵, R^{3'}, R^{4'}, and R^{5'} each independently represent a hydrogen atom, or a chained hydrocarbon group having 1 to 30 carbon atoms which may have a branch or an unsaturated bond (here, in a case where X¹, X², and X³ each independently represent an oxygen atom, corresponding R^{3'}, R^{4'}, or R^{5'} does not exist).

In a case where any one of X¹ to X³ is a nitrogen atom, -CONR^{m}R^{m'} (m and m' represent the same number, which is any one of 3, 4, or 5 in correspondence with X¹ to X³) represents a substituted or unsubstituted amido group, and in a case where any one of X¹ to X³ is an oxygen atom, -COOR^{m} (m represents any number of 3, 4, or 5 in correspondence with X¹ to X³) represents a carboxyl group or an ester group.

Having a condition to satisfy the above described proviso, R⁴, R⁵, R^{3'}, R^{4'}, and R^{5'} each independently represent a hydrogen atom, or a chained hydrocarbon group having 1 to 30 carbon atoms, preferably 8 to 24 carbon atoms, more preferably 14 to 22 carbon atoms, which may have a branch or an unsaturated bond, further preferably represent a hydrogen atom or a chained saturated hydrocarbon group having 14 to 22 carbon atoms which may have a branch, and may also represent a saccharide residue derived from monosaccharide and polysaccharide.

As specific examples, R⁴, R⁵, R^{3'}, R^{4'}, and R^{5'} each independently represent any one of a hydrogen atom, a methyl group, an ethyl group, a propyl group, an isopropyl group, a butyl group, a 1-methylpropyl group, a 2-methylpropyl group, a pentyl group, a 1-methylbutyl group, a 2-methylbutyl group, a 3-methylbutyl group, a 1-ethyl propyl group, a hexyl group, a 1-methylpentyl group, a 2-methylpentyl group, a 3-methylpentyl group, a 4-methylpentyl group, a 1-ethylbutyl group, a 2-ethylbutyl group, a heptyl group, a 1-methylhexyl group, a 2-methylhexyl group, a 3-methylhexyl group, a 4-methylhexyl group, a 5-methylhexyl group, a 1-ethylpentyl group, a 2-ethylpentyl group, a 3-ethylpentyl group, an octyl group, a 1-methylheptyl group, a 2-methylheptyl group, a 3-methylheptyl group, a 4-methylheptyl group, a 5-methylheptyl group, a 6-methylheptyl group, a 1-ethylhexyl group, a 2-ethylhexyl group, a 3-ethylhexyl group, a 4-ethylhexyl group, a 1-propylpentyl group, a 2-propylpentyl group, a nonyl group, a decyl group, a undecyl group, a 10-undecenyl group, a dodecyl group, a tridecyl group, a tetradecyl group, a pentadecyl group, a hexadecyl group, a 9-hexadecenyl group, a heptadecyl group, an octadecyl group, an isostearyl group, a cis-9-octadecenyl group, a 11-octadecenyl group, a cis,cis-9,12-octadecadienyl group, a9,12,15-octadecatrienyl group, a 6,9,12-octadecatrienyl group, a 9,11,13-octadecatrienyl group, a nonadecyl group, a 2,6,10,14-tetramethylpentadecyl group, an icosanyl group, a 8,11-icosadienyl group, a 5,8,11-icosatrienyl group, a 5,8,11,14-icosatetraenyl group, a 3,7,11,15-tetramethylhexadecyl group, a henicosanyl group, a docosanyl group, and the like.

Having a condition to satisfy the above described proviso, preferable examples among these include an embodiment in which R⁴, R⁵, R^{3'}, R^{4'}, and R^{5'} each independently represent any one of a hydrogen atom, a methyl group, an ethyl group, a propyl group, an isopropyl group, a butyl group, a hexyl group, an octyl group, a decyl group, a undecyl group, a dodecyl group, a tridecyl group, a tetradecyl group, a pentadecyl group, a hexadecyl group, a heptadecyl group, an octadecyl group, and an isostearyl group.

More preferable examples include an embodiment in which one or two of R³, R⁴, R⁵, R^{3'}, R^{4'}, and R^{5'} represent any one of a methyl group, an ethyl group, a propyl group, an isopropyl group, a butyl group, a hexyl group, an octyl group, a decyl group, a undecyl group, a dodecyl group, a tridecyl group, a tetradecyl group, a pentadecyl group, a hexadecyl group, a heptadecyl group, an octadecyl group, and an isostearyl group, and the rest represent hydrogen atoms.

X¹ to X³ each independently represent a nitrogen or an oxygen atom, and all may represent the same atoms, or atoms may be mixed, and a preferable example is the same atom, and more preferable example is an oxygen atom.

In a case where X¹ to X³ all represent oxygen atoms, a modified site of a carboxyl group is -COOR^{m} (m represents any number of 3, 4, or 5 in correspondence with X¹ to X³), and represents a carboxyl group or an ester group. In this case, R^{3'}, R^{4'}, or R^{5'} does not exist.

Preferable examples of the hydroxycitric acid derivative according to the agent for improving mitochondrial function of the present invention include a compound of which at least one of hydroxyl groups at the 2-position or the 3-position of hydroxycitric acid is modified, and a compound of which at least one of carboxyl groups of the compound is further modified.

In addition, examples of a derivative in which a carboxyl group in a molecule is modified include a compound of which at least one site of a carboxyl group of the above described derivative of which a hydroxyl group in a molecule is modified, is a substituted or unsubstituted amido group, or an ester group.

In other words, examples of the hydroxycitric acid derivative according to the agent for improving mitochondrial function of the present invention include a compound of which at least one of hydroxyl groups is modified or a carboxyl group is not modified as described above, and a compound of which at least one of hydroxyl groups and at least one of carboxyl groups are modified as described above. Specifically, preferable examples of these compounds include a compound in which the above examples of R¹, R², X¹ to X³, R³, R⁴, R⁵, R^{3'}, R^{4'}, and R^{5'} are combined.

As described above, it is possible to modify a functional group in a molecule into various combinations, and particularly preferable examples include a compound represented by the following Formula (IIa) of which R² represents a hydrogen atom, R³ to R⁵ all represent hydrogen atoms, and X¹ to X³ all represent oxygen atoms, or a compound represented by the following Formula (IIb) of which R² represents a hydrogen atom, R³ to R⁵ each independently represent a hydrogen atom, or a chained hydrocarbon group having 1 to 30 carbon atoms which may have a branch or an unsaturated bond (here, R³ to R⁵ do not represent hydrogen atoms at the same time), and X¹ to X³ all represent oxygen atoms.

In this case, that is, in the case where the hydroxycitric acid derivative according to the agent for improving mitochondrial function of the present invention is a compound represented by the following Formula (IIa) or a compound represented by the following Formula (IIb), R¹ represents any one group selected from the group represented by Formula (Ia), and among the groups represented by Formula (Ia), R¹ preferably represents the group represented by Formula (Ia-1). In addition, in Formula (Ia), R⁶ preferably represents a chained hydrocarbon group having 7 to 23 carbon atoms, preferably a chained hydrocarbon group having 10 to 22 carbon atoms, more preferably a chained hydrocarbon group having 13 to 21 carbon atoms, and further more preferably a chained hydrocarbon group having 13 to 18 carbon atoms, which may have a branch, an unsaturated bond, or a substituent.

It is preferable that a chained hydrocarbon group be a chained saturated hydrocarbon group, and it is more preferable that a chained hydrocarbon group not have a substituent. R⁶ preferably represents a chained saturated hydrocarbon group having 7 to 23 carbon atoms which does not have a substituent, preferably a chained saturated hydrocarbon group having 10 to 22 carbon atoms which does not have a substituent, more preferably a chained saturated hydrocarbon group having 13 to 21 carbon atoms which does not have a substituent, and further more preferably a chained saturated hydrocarbon group having 13 to 18 carbon atoms which does not have a substituent.

It is preferable that a chained hydrocarbon group be a linear saturated hydrocarbon group, and it is more preferable that a chained hydrocarbon group not have a substituent. R⁶ preferably represents a linear saturated hydrocarbon group having 7 to 23 carbon atoms which does not have a substituent, preferably a linear saturated hydrocarbon group having 10 to 22 carbon atoms which does not have a substituent, more preferably a linear saturated hydrocarbon group having 13 to 21 carbon atoms which does not have a substituent, and further more preferably a linear saturated hydrocarbon group having 13 to 18 carbon atoms which does not have a substituent.

It is desirable that R¹ represent an acyl group having 14 to 22 carbon atoms.

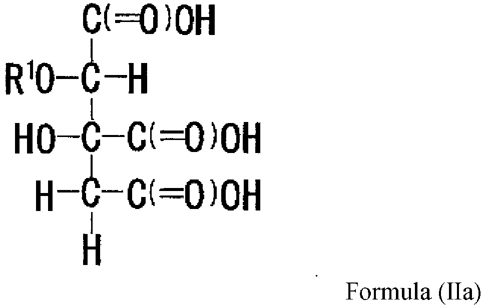

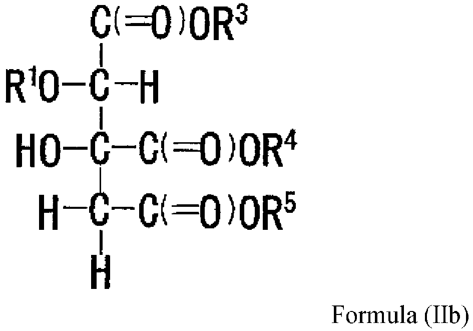

Specific examples of the hydroxycitric acid derivative according to the agent for improving mitochondrial function of the present invention include hydroxycitric acid-2-octanoate, hydroxycitric acid-2-caprate, hydroxycitric acid-2-laurate, hydroxycitric acid-2-myristate, hydroxycitric acid-2-palmitate, hydroxycitric acid-2-stearate, hydroxycitric acid-2-behenoate, hydroxycitric acid-2-isopalmitate, hydroxycitric acid-2-isostearate, hydroxycitric acid-2-hexyl decanoate, hydroxycitric acid-2-linoleate, hydroxycitric acid monomethyl ester-2-myristate, hydroxycitric acid monomethyl ester-2-palmitate, hydroxycitric acid monomethyl ester-2-stearate, and the like.

Among these, preferable examples include hydroxycitric acid-2-laurate, hydroxycitric acid-2-myristate, hydroxycitric acid-2-palmitate, hydroxycitric acid-2-stearate, hydroxycitric acid-2-behenoate, hydroxycitric acid-2-isopalmitate, hydroxycitric acid-2-isostearate, hydroxycitric acid-2-hexyl decanoate, and hydroxycitric acid-2-linoleate.

More preferable examples include hydroxycitric acid-2-myristate, hydroxycitric acid-2-palmitate, and hydroxycitric acid-2-stearate.

Examples of a salt of the hydroxycitric acid derivative according to the agent for improving mitochondrial function of the present invention include an alkali metal salt and an alkaline earth metal salt of the above hydroxycitric acid derivative.

Examples of the alkali metal salt include a sodium salt, a potassium salt, and the like and examples of the alkaline earth metal salt include a calcium salt, and the like.

The agent for improving mitochondrial function of the present invention exhibits effect on improving mitochondrial function in cells, which is considered to be achieved by the fact that the agent for improving mitochondrial function of the present invention suppresses apoptosis induced by the degradation of the mitochondrial function.

Those skilled in the art can select a method for administering the hydroxycitric acid derivative or a salt thereof according to the agent for improving mitochondrial function of the present invention, from known methods such as oral administration, intraarterial injection, and intravenous injection. The hydroxycitric acid derivative or a salt thereof according to the agent for improving mitochondrial function of the present invention can be used by being added to a medium of cells to be cultured in a case of administering to cells to be cultured or the like.

The dose of the above hydroxycitric acid derivative or a salt thereof may be determined according to a blood concentration or a concentration in culture solution of the hydroxycitric acid derivative or a salt thereof. For example, as a blood concentration or a concentration in culture solution (molar ratio) of the hydroxycitric acid derivative or a salt thereof, approximately 0.001 µM to 100 µM is preferable, approximately 0.005 µM to 50 µM is more preferable, and approximately 0.005 µM to 20 µM is further preferable.

In another embodiment, the present invention provides a method for improving mitochondrial function, comprising bringing an effective amount of a hydroxycitric acid derivative or a salt thereof into contact with cells to be cultured.

In still another embodiment, the present invention provides a hydroxycitric acid derivative or a salt thereof for preventing or treating diseases related to the degradation of mitochondrial function.

In still further another embodiment, the present invention provides the use of a hydroxycitric acid derivative or a salt thereof for preventing or treating diseases related to the degradation of mitochondrial function.

In still further another embodiment, the present invention provides the use of a hydroxycitric acid derivative or a salt thereof for producing a preventive or therapeutic agent for diseases related to the degradation of mitochondrial function.

In still further another embodiment, the present invention provides a method for preventing or treating diseases related to the degradation of mitochondrial function, the method comprising administering an effective amount of a hydroxycitric acid derivative or a salt thereof to a patient in need of treatment.

Examples of diseases related to the degradation of mitochondrial function include mitochondrial disorders, mitochondrial diseases, and the like.

The improvement in the mitochondrial function can be confirmed by measuring cells in which apoptosis had been caused using a method described in Example described below with a mitochondrial membrane potential as an index. For example, a cell group brought into contact with the agent for improving mitochondrial function and a cell group not brought into contact with the agent for improving mitochondrial function are compared, and in a case where a ratio of cells in which apoptosis had been caused, which is obtained using a mitochondrial membrane potential as an index, is lower in the cell group brought into contact with the agent for improving mitochondrial function, it can be determined that the agent for improving mitochondrial function has effects on improving mitochondrial function.

The agent for improving mitochondrial function may be provided as a hydroxycitric acid derivative or a salt thereof as it is, or may be provided as a composition which is mixed with an appropriate additive. Examples of the additive include an oily base described below, a moisturizing agent, a touch improving agent, a surfactant, a polymer, a thickening-gelling agent, a solvent, a propellant, an antioxidant, a reductant, an oxidant, a preservative, an antibacterial agent, a chelating agent, a pH adjusting agent, an acid, an alkali, a powder, an inorganic salt, an ultraviolet absorbent, a whitening agent, vitamins and derivatives thereof, an antiphlogistic agent, an antiinflammatory agent, a drug for hair growth, a blood circulation accelerator, a stimulant, hormone, an antiwrinkle agent, an antiaging agent, a tightening agent, a cold sense agent, a warm sense agent, a wound healing accelerator, an irritation alleviating agent, an analgesic, a cell activator, plant, animal, and microbial extract, an antipruritic agent, a keratin releasing-dissolving agent, an antiperspirant, a cooling agent, an astringent, enzyme, nucleic acid, fragrance, coloring matter, a colorant, a dye, a pigment, water, a metal-containing compound, an unsaturated monomer, polyhydric alcohol, a polymer additive, an antiinflammatory analgesic agent, an antifungal agent, an antihistamine, a hypnotic sedative, a tranquilizer, an antihypertensive agent, a hypotensive diuretic, an antibiotic, an anesthetic, an antibacterial substance, an antiepileptic agent, a coronary vasodilator, an herbal medicine, an auxiliary agent, a wetting agent, an astringent, a thickening agent, a tackifying material, an antipruritic agent, a keratin layer softening and peeling agent, an oily material, an ultraviolet screening agent, an antiseptic preservative, an antioxidant, a liquid matrix, fat-soluble material, and polymer carboxylate, an additive, a metal soap, and the like.

### (Method for Producing Hydroxycitric Acid Derivative and Salt Thereof)

Next, a method for producing a hydroxycitric acid derivative or a salt thereof according to the agent for improving mitochondrial function of the present invention will be described.

The method for producing a hydroxycitric acid derivative or a salt thereof according to the agent for improving mitochondrial function of the present invention is not particularly limited, and it is possible to produce the hydroxycitric acid derivative or a salt thereof by reacting hydroxycitric acid and/or an alkali metal salt thereof and/or an alkaline earth metal salt thereof with a carboxylic acid derivative, a phosphoric acid derivative or a sulfonic acid derivative in an appropriate solvent.

Examples of the method using a commercially available hydroxycitric acid as a raw material are as follows.
(1) A method of directly esterifying a hydroxyl group of hydroxycitric acid
(2) A method of esterifying a hydroxyl group after esterifying a carboxyl group of hydroxycitric acid, and cleaving the ester bond moiety to convert to a carboxyl group
(3) A method of esterifying a hydroxyl group after esterifying a carboxyl group of hydroxycitric acid
(4) A method of esterifying a carboxyl group after esterifying a hydroxyl group of hydroxycitric acid
(5) A method of esterifying a hydroxyl group after amidating a carboxyl group of hydroxycitric acid
(6) A method of esterifying a hydroxyl group after amidating a carboxyl group of hydroxycitric acid, and cleaving the amide bond moiety to convert to a carboxyl group
(7) A method of amidating a carboxyl group after esterifying a hydroxyl group of hydroxycitric acid

In addition, there is no particular limitation on a method for producing a compound which is preferable among compounds according to the agent for improving mitochondrial function of the present invention, the compound being represented by Formula (IIa) in which R¹ represents an acyl group having 14 to 22 carbon atoms, R² represents a hydrogen atom, R³ to R⁵ all represent hydrogen atoms, and X¹ to X³ all represent oxygen atoms. For example, the production is possible by the above method (1) or (2) using a commercially available hydroxycitric acid as a raw material.

In the case of the above production method (1), an ester of hydroxycitric acid is obtained by esterifying hydroxycitric acid (or an alkali metal salt thereof or an alkaline earth metal salt thereof) according to a commonly used method. More specifically, for example, it is possible to produce long-chain acyl hydroxycitrate by reacting a corresponding long-chain carboxylic acid with hydroxycitric acid (an alkali metal salt or an alkaline earth metal salt of hydroxycitric acid may be used) in an appropriate solvent in the presence of a catalyst (for example, Tetrahedron Letters, pg. 4011, 1970). Alternatively, the production is possible by reacting a corresponding long-chain acyl chloride with hydroxycitric acid (an alkali metal salt or an alkaline earth metal salt of hydroxycitric acid may be used) under the environment of an appropriate solvent (for example, Chem. Rev., Vol. 52, pg. 239, 1953). Still alternatively, the production is possible by using a corresponding long-chain carboxylic acid anhydride and hydroxycitric acid (an alkali metal salt or an alkaline earth metal salt of hydroxycitric acid may be used) (for example, Organic Synthesis, Vol. 4, pg. 560). Further alternatively, the production is possible by using various esterifying agents capable of activating a corresponding long-chain carboxylic acid, and hydroxycitric acid (an alkali metal salt or an alkaline earth metal salt of hydroxycitric acid may be used) (for example, Organic Synthesis, Vol. 63, pg. 183). Still further alternatively, the production is possible by using a corresponding long-chain alkyl nitrile and hydroxycitric acid (an alkali metal salt or an alkaline earth metal salt of hydroxycitric acid may be used) (for example, Organic Synthesis, Vol. 1, pg. 27).

In addition, more specifically, in the case of the above production method (2), the formation is possible by performing, for example, a first step in which hydroxycitric acid (an alkali metal salt or an alkaline earth metal salt of hydroxycitric acid may be used) is reacted with an alcohol (such as benzyl alcohol) in an appropriate solvent to temporarily protect (for example, protect with benzyl ester) a tri-carboxylic acid moiety of hydroxycitric acid, a second step in which a hydroxyl group of the compound obtained in the first step is esterified by any one of the methods described above, and a third step in which ester moieties formed in the first step of the compound obtained in the second step are deprotected (cleaving ester bond moieties).

The alcohols used in the first step are not particularly limited as long as selective protection and deprotection of only the carboxyl group moieties of hydroxycitric acid are possible. Examples thereof include benzyl alcohol, substituted benzyl alcohol, tert-butanol, 2,2,2-trichloroethanol, and the like. Among these, benzyl alcohol and substituted benzyl alcohol are preferable. In a case of protecting with benzyl ester in the first step, deprotection is possible by a reaction such as catalytic reduction in the third step.

In addition, there is no particular limitation on a method for producing a compound which is preferable among compounds according to the agent for improving mitochondrial function of the present invention, the compound being represented by Formula (IIb) in which R¹ represents an acyl group having 14 to 22 carbon atoms, R² represents a hydrogen atom, R³ to R⁵ each independently represent a hydrogen atom, or a chained hydrocarbon group having 1 to 30 carbon atoms which may have a branch or an unsaturated bond (here, R³ to R⁵ do not represent hydrogen atoms at the same time), and X¹ to X³ all represent an oxygen atom. For example, the production is possible by the above methods (2) to (4) using a commercially available hydroxycitric acid as a raw material.

In this case, in the above production method (2), the production in the third step of deprotection is possible by deprotecting (cleaving a part of ester bond moieties) a part of ester moieties formed in the first step of the compound obtained in the second step.

As long as the method for producing the hydroxycitric acid derivative or a salt thereof according to the agent for improving mitochondrial function (the above production method (2)) comprises the above three steps, other steps may be included therebetween. For example, steps such as distillation, extraction or crystallization for separating and purifying products may be included between the first step and the second step, or the second step and the third step.

In these production methods, it is also possible to modify only the hydroxyl group at the 2-position of hydroxycitric acid by appropriately adjusting an amount of use of a reagent and using the difference in reactivity between the hydroxyl group at the 2-position of hydroxycitric acid and the hydroxyl group at the 3-position of hydroxycitric acid.

In a case of using hydroxycitric acid, an alkali metal salt or an alkaline earth metal salt thereof as a starting material, a solvent to be used is not particularly limited as long as a solvent can dissolve or suspend these starting materials. Polar solvents such as dimethylformamide (hereinafter will be also referred to as "DMF"), dimethylsulfoxide, trisdimethylaminophosphine, and water are preferable. These solvents can be used alone or as a mixture of two or more.

In a case of using hydroxycitrate ester or amide as a starting material, a solvent to be used is not particularly limited as long as a solvent can dissolve or suspend these starting materials. Tetrahydrofuran (hereinafter will be also referred to as "THF"), chloroform, dichloromethane, dioxane, and the like are preferable. These solvents can be used alone or as a mixture of two or more.

A reaction temperature is not particularly limited as long as it is equal to or lower than a boiling point of a solvent to be used. Specifically, for example, the range of -20°C to 100°C is preferable, and the range of 0°C to 60°C is more preferable.

A reaction concentration is not particularly limited, and the range of 0.0001 mol/dm³ to 10 mol/dm³ is preferable, and the range of 0.1 mol/dm³ to 1 mol/dm³ is more preferable.

Since hydroxycitric acid gives a cyclized product under acidic conditions, it is desirable that a reaction system be alkaline.

In addition, it is possible to easily produce a salt of the hydroxycitric acid derivative by performing such pH adjustment. For example, it is possible to obtain a Na salt of the hydroxycitric acid derivative if NaOH is used as a pH adjusting agent.

After completion of the reaction, purification can be performed by a known method such as recrystallization or silica gel column chromatography.

As one embodiment, the agent for improving mitochondrial function of the present invention may be an agent in which a hydroxycitric acid derivative or a salt thereof is produced by reacting hydroxycitric acid and/or an alkali metal salt thereof and/or an alkaline earth metal salt thereof with a carboxylic acid derivative, a phosphoric acid derivative or a sulfonic acid derivative in a solvent.

As one embodiment, the agent for improving mitochondrial function of the present invention may be an agent produced by a method comprising producing a hydroxycitric acid derivative or a salt thereof by a first step of producing a hydroxycitric acid (tri)ester by reacting hydroxycitric acid and/or an alkali metal salt thereof and/or an alkaline earth metal salt thereof with alcohol in a solvent, a second step of esterifying a hydroxyl group of the compound obtained in the first step by reacting an aliphatic carboxylic acid derivative and the compound obtained in the first step, and a third step of cleaving a part or all of the ester bond moieties formed in the first step from the ester bond moieties of the compound obtained in the second step.

### «Anti-aging Composition»

An anti-aging composition of the present invention includes a hydroxycitric acid derivative represented by the following Formula (I) or a salt thereof, as an active ingredient 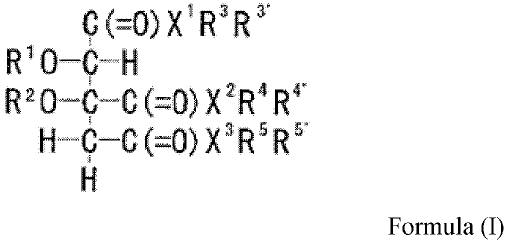 (in Formula (I), R¹ and R² each independently represent a hydrogen atom or any one group selected from the group represented by the following Formula (Ia) (here, R¹ and R² do not represent hydrogen atoms at the same time), X¹ to X³ each independently represent a nitrogen or an oxygen atom, and R³, R⁴, R⁵, R^{3'}, R^{4'}, and R^{5'} each independently represent a hydrogen atom, or a chained hydrocarbon group having 1 to 30 carbon atoms which may have a branch or an unsaturated bond (here, in a case where X¹, X², and X³ each independently represent an oxygen atom, corresponding R^{3'}, R^{4'}, or R^{5'} does not exist), 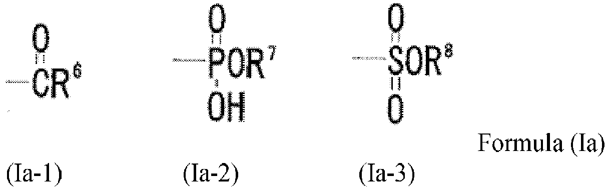 (in Formula (Ia), R⁶ to R⁸ each independently represent a hydrogen atom, an aryl group, or a chained hydrocarbon group having 1 to 30 carbon atoms which may have a branch, an unsaturated bond, or a substituent).

A hydroxycitric acid derivative or a salt thereof of the anti-aging composition is the same as exemplified in the above agent for improving mitochondrial function, and therefore the explanation thereof will be omitted.

Since the hydroxycitric acid derivative represented by the following Formula (I) or a salt thereof is contained in the above agent for improving mitochondrial function, the anti-aging composition of the present invention may contain the above agent for improving mitochondrial function, as an active ingredient.

The anti-aging composition of the present invention may be provided as a hydroxycitric acid derivative or a salt thereof as it is, or may contain components normally used for a skin external agent such as a pharmacologically acceptable carrier as a skin external agent or an additive as necessary in the scope of not harming the effect of the present invention. Examples of the components include an oily base, a moisturizing agent, a touch-improving agent, a surfactant, a polymer, a thickening-gelling agent, a solvent, a propellant, an antioxidant, a reductant, an oxidant, a preservative, an antibacterial agent, a chelating agent, a pH-adjusting agent, an acid, an alkali, a powder, an inorganic salt, an ultraviolet absorbent, a whitening agent, vitamins and derivatives thereof, an antiphlogistic agent, an antiinflammatory agent, a drug for hair growth, a blood circulation accelerator, a stimulant, hormone, an antiwrinkle agent, an antiaging agent, a tightening agent, a cold sense agent, a warm sense agent, a wound healing accelerator, an irritation alleviating agent, an analgesic, a cell activator, plant, animal, and microbial extract, an antipruritic agent, a keratin releasing-dissolving agent, an antiperspirant, a cooling agent, an astringent, enzyme, nucleic acid, fragrance, coloring matter, a colorant, a dye, a pigment, water, a metal-containing compound, an unsaturated monomer, polyhydric alcohol, a polymer additive, an antiinflammatory analgesic agent, an antifungal agent, an antihistamine, a hypnotic sedative, a tranquilizer, an antihypertensive agent, a hypotensive diuretic, an antibiotic, an anesthetic, an antibacterial substance, an antiepileptic agent, a coronary vasodilator, an herbal medicine, an auxiliary agent, a wetting agent, an astringent, a thickening agent, a tackifying material, an antipruritic agent, a keratin layer softening and peeling agent, an oily material, an ultraviolet screening agent, an antiseptic preservative, an antioxidant, a liquid matrix, fat-soluble material, and polymer carboxylate, an additive, and a metal soap.

Examples of the oily base include higher alcohols such as cetanol, myristyl alcohol, oleyl alcohol, lauryl alcohol, cetostearyl alcohol, stearyl alcohol, arachyl alcohol, behenyl alcohol, jojoba alcohol, chimyl alcohol, selachyl alcohol, batyl alcohol, hexyldecanol, isostearyl alcohol, 2-octyl dodecanol, a dimer diol; aralkyl alcohols such as benzyl alcohol and derivatives thereof; higher fatty acids such as lauric acid, myristic acid, palmitic acid, stearic acid, isostearic acid, behenic acid, undecylenic acid, 12-hydroxystearic acid, palmitoleic acid, oleic acid, linolic acid, linolenic acid, erucic acid, docosahexaenoic acid, eicosapentaenoic acid, isohexadecanoic acid, anteheneicosanoic acid, a long-chain branched fatty acid, a dimer acid, and a hydrogenated dimer acid, and metal soaps such as an aluminum salts thereof, a calcium salt thereof, a magnesium salt thereof, a zinc salt thereof, and a potassium salt thereof, and nitrogen-containing derivatives such as amide; hydrocarbons such as liquid paraffin (mineral oil), heavy liquid isoparaffin, light liquid isoparaffin, an α-olefin oligomer, polyisobutene, hydrogenated polyisobutene, polybutene, squalane, olive-derived squalane, squalene, vaseline, and solid paraffin; waxes such as candelilla wax, carnauba wax, rice wax, Japan wax, beeswax, montan wax, ozokerite, ceresine, paraffin wax, microcrystalline wax, petrolatum, Fischer-Tropsch wax, polyethylene wax, and an ethylene-propylene copolymer; vegetable fats and oils such as coconut oil, palm oil, palm kernel oil, safflower oil, olive oil, castor oil, avocado oil, sesame oil, tea oil, evening primrose oil, wheat germ oil, macadamia nut oil, hazelnut oil, kukui nut oil, rose hip oil, meadowfoam oil, persic oil, tea tree oil, peppermint oil, corn oil, rapeseed oil, sunflower oil, wheat germ oil, linseed oil, cottonseed oil, soybean oil, peanut oil, rice bran oil, cacao oil, shea oil, hydrogenated coconut oil, hydrogenated castor oil, jojoba oil, and hydrogenated jojoba oil; animal fats and oils such as beef tallow, milk fat, horse fat, egg yolk oil, mink oil, and turtle oil; animal waxes such as spermacetieti, lanolin, and orange roughy oil; lanolins such as liquid lanolin, reduced lanolin, adsorption-purified lanolin, lanolin acetate, liquid lanolin acetate, hydroxy lanolin, polyoxyethylene lanolin, lanolin fatty acid, hard lanoline fatty acid, lanolin alcohol, acetylated lanolin alcohol, and acetic acid (cetyl/lanolyl) ester; phospholipids such as lecithin, phosphatidylcholine, phosphatidylethanolamine, phosphatidylserine, phosphatidylglycerol, phosphatidylinositol, sphingophospholipids such as sphingomyelin, phosphatidic acid, and lysolecithin; phospholipid derivatives such as hydrogenated soybean phospholipid, partially hydrogenated soybean phospholipid, hydrogenated egg yolk phospholipid, and partially hydrogenated egg yolk phospholipid; sterols such as cholesterol, dihydrocholesterol, lanosterol, dihydrolanosterol, phytosterol, and cholic acid; sapogenins; saponins; sterol esters such as cholesteryl acetate, cholesteryl nonanoate, cholesteryl stearate, cholesteryl isostearate, cholesteryl oleate, di(cholesteryl/behenyl/octyldodecyl) N-lauroyl-L-glutamate, di(cholesteryl/octyldodecyl) N-lauroyl-L-glutamate, di(phytosteryl/behenyl/octyldodecyl) N-lauroyl-L-glutamate, di(phytosteryl/octyldodecyl) N-lauroyl-L-glutamate, acylsarcosine alkyl esters such as isopropyl N-lauroyl sarcosinate, cholesteryl 12-hydroxystearate, cholesteryl macadamiate, phytosteryl macadamiate, phytosteryl isostearate, soft cholesteryl lanolin, hard cholesteryl lanolin, long-chain branched fatty acid cholesterol, and long-chain α-hydroxyfatty acid cholesterol; lipid complexes such as a phospholipid-cholesterol complex and a phospholipid-phytosterol complex; monoalcohol-carboxylic acid esters such as octyldodecyl myristate, hexyldecyl myristate, octyldodecyl isostearate, cetyl palmitate, octyl dodecyl palmitate, cetyl octanoate, hexyldecyl octanoate, isotridecyl isononanoate, isononyl isononanoate, octyl isononanoate, isotridecyl isononanoate, isodecyl neopentanoate, isotridecyl neopentanoate, isostearyl neopentanoate, octyldodecyl neodecanoate, oleyl oleate, octyldodecyl oleate, octyldodecyl ricinoleate, octyldodecyl lanolate, hexyldecyl dimethyl octanoate, octyldodecyl erucate, hard castor oil isostearate, ethyl oleate, ethyl avocadate, isopropyl myristate, isopropyl palmitate, octyl palmitate, isopropyl isostearate, isopropyl lanolate, diethyl sebacate, diisopropyl sebacate, dioctyl sebacate, diisopropyl adipate, dibutyloctyl sebacate, diisobutyl adipate, dioctyl succinate, and triethyl citrate; oxyacid esters such as cetyl lactate, diisostearyl malate, and hydrogenated castor oil monoisostearate; polyhydric alcohol fatty acid esters such as; glyceryl trioctanoate, glyceryl trioleate, glyceryl triisostearate, glyceryl diisostearate, glycerol tri- (caprylate/capriate), glycerol tri-(caprylate/caprinate/myristate/stearate), hydrogenated rosin triglyceride (hydrogenated ester gum), rosin triglyceride (ester gum), glyceryl behenate/eicosadioate, trimethylol propane trioctanoate, trimethylol propane triisostearate, neopentyl glycol dioctanoate, neopentyl glycol dicaprate, 2-butyl-2-ethyl-1,3-propanediol dioctanoate, propylene glycol dioleate, pentaerythrityl tetraoctanoate, hydrogenated pentaerythrityl rosinate, ditrimethylol propane triethyl hexaneoate, ditrimethylol propane (isostearate/sebacate), pentaerythrityl triethyl hexanoate, dipentaerythrityl (hydroxystearate/stearate/rosinate), diglyceryl diisostearate, polyglyceryl tetraisostearate, polyglyceryl-10 nonaisostearate, polyglyceryl-8 deca(erucate/isostearate/ricinoleate), (hexyldecanoic acid/sebacic acid) diglyceryl oligoester, and glycol distearate (ethylene glycol distearate); derivatives of dimer acids or dimer diols such as diisopropyl dimer dilinoleate, diisostearyl dimer dilinoleate, di(isostearyl/phytosteryl) dimer dilinoleate, (phytosteryl/behenyl) dimer dilinoleate, (phytosteryl/isostearyl/cetyl/stearyl/behenyl) dimer dilinoleate, dimer dilinoleyl dilinoleate, dimer dilinoleyl diisostearate, dimer dilinoleyl hydrogenated rosin condensate, dimer linoleic acid hard castor oil, and hydroxyalkyl dimer dilinoleyl ether; fatty acid alkanolamides such as; coconut oil fatty acid monoethanolamide (cocamide MEA), coconut oil fatty acid diethanolamide (cocamide DEA), lauric acid monoethanolamide (lauramide MEA), lauric acid diethanolamide (lauramide DEA), lauric acid monoisopropanolamide (lauramide MIPA), palmitic acid monoethanolamide (palmitamide MEA), palmitic acid diethanolamide (palmitamide DEA), and coconut oil fatty acid methylethanolamide (cocamide methyl MEA); silicones including amino-modified silicones such as dimethicone (dimethyl polysiloxane), highly polymerized dimethicone (highly polymerized dimethyl polysiloxane), cyclomethicone (cyclic dimethyl siloxane, decamethyl cyclopentasiloxane), phenyl trimethicone, diphenyl dimethicone, phenyl dimethicone, stearoxy propyl dimethyl amine, a (aminoethyl aminopropyl methicone/dimethicone) copolymer, dimethiconol, a dimethiconol cross polymer, a silicone resin, silicone rubber, aminopropyl dimethicone, and amodimethicone, polyether-modified silicones such as cation-modified silicone and dimethicone copolyol, polyglycerin-modified silicone, sugar-modified silicone, carboxylic acid-modified silicone, phosphoric acid-modified silicone, sulfuric acid-modified silicone, alkyl-modified silicone, fatty acid-modified silicone, alkyl ether-modified silicone, amino acid-modified silicone, peptide-modified silicone, fluorine-modified silicone, cation-modified and polyether-modified silicone, amino-modified and polyether-modified silicone, alkyl-modified and polyether-modified silicone, and a polysiloxane-oxyalkylene copolymer; and fluorine-based oils such as perfluorodecane, perfluorooctane, and perfluoro polyether.

Examples of the moisturizing agent and the touch-improving agent include polyhydric alcohols such as glycerin, propylene glycol, 1,3-butylene glycol, sorbitol, polyglycerin, polyethylene glycol, and dipropylene glycol; water-soluble polymer such as NMF components such as sodium lactate, hyaluronic acid, collagen, mucopolysaccharides, and chondroitin sulfate; polyols such as glycerin, 1,3-butylene glycol, propylene glycol, 3-methyl-1,3-butanediol, 1,3-propanediol, 2-methyl-1,3-propanediol, trimethylol propane, pentaerythritol, hexylene glycol, diglycerin, polyglycerin, diethylene glycol, polyethylene glycol, dipropylene glycol, polypropylene glycol, and an ethylene glycol-propylene glycol copolymer, and polymers thereof; glycol alkyl ethers such as diethylene glycol monoethyl ether (ethoxydiglycol), ethylene glycol monoethyl ether, ethylene glycol monobutyl ether, and diethylene glycol dibutyl ether; sugar alcohols such as sorbitol, xylitol, erythritol, mannitol, and maltitol; saccharides such as glucose, fructose, galactose, mannose, threose, xylose, arabinose, fucose, ribose, deoxyribose, maltose, trehalose, lactose, raffinose, gluconic acid, glucuronic acid, cyclodextrins (α-, β-, and γ-cyclodextrins, and cyclodextrins obtained by modification such as maltosylation, hydroxyalkylation, and the like), glucan, chitin, chitosan, heparin and derivatives thereof, pectin, arabinogalactan β, dextrin, dextran, glycogen, ethyl glucoside, and a glucosyl ethyl methacrylate polymer or a copolymer, and derivatives thereof; hyaluronic acid and sodium hyaluronate; sodium chondroitin sulfate; mucoitinsulfuric acid, charonin sulfate, keratosulfate, dermatan sulfate; snow fungus extract, and snow fungus polysaccharide; fucoidan; tuberosa polysaccharide, and naturally derived polysaccharide; organic acids such as citric acid, tartaric acid, and lactic acid, and salts thereof; urea; 2-pyrrolidone-5-carboxylic acid and salts thereof such as a sodium salt thereof; amino acids such as betaine (trimethyl glycine), proline, hydroxyproline, arginine, lysine, serine, glycine, alanine, phenylalanine, tyrosine, β-alanine, threonine, glutamic acid, glutamine, asparagine, aspartic acid, cysteine, cystine, methionine, leucine, isoleucine, valine, tryptophan, histidine, and taurine, and salts thereof; protein peptides such as collagen, fish-derived collagen, atelocollagen, gelatin, elastin, collagen decomposed peptide, hydrolyzed collagen, hydroxypropyl ammonium chloride hydrolyzed collagen, elastin decomposed peptide, keratin decomposed peptide, hydrolyzed keratin, conchiolin decomposed peptide, hydrolyzed conchiolin, silk proteolytic peptide, hydrolyzed silk, lauroyl hydrolyzed silk sodium, soybean proteolytic peptide, wheat proteolytic peptide, hydrolyzed wheat protein, casein decomposed protein, and acylated peptide, and derivatives thereof; acylated peptides such as palmitoyl oligopeptide, palmitoyl pentapeptide, and palmitoyl tetrapeptide; silylated peptides; animal-plant extract components such as a lactic acid bacteria culture solution, yeast extract, egg shell membrane protein, bovine submaxillary gland mucin, hypotaurine, sesame lignan glycoside, glutathione, albumin, whey, choline chloride, phosphoryl choline; placenta extract, elastin, collagen, aloe extract, hamamelis water, loofah water, chamomilla extract, licorice extract, comfrey extract, silk extract, rosa roxburghii extract, yarrow extract, eucalyptus extract, and melilot extract, ceramides such as natural type ceramides (types 1, 2, 3, 4, 5, and 6), hydroxyceramide, pseudo-ceramide, sphingoglycolipid, and ceramide- and sugar ceramide-containing extract.

Examples of the surfactant include anionic surfactants such as a lauryl sulfuric acid ester salt, polyoxyethylene alkyl ether sulfate, an alkyl benzene sulfonate, polyoxyethylene alkyl ether phosphoric acid, polyoxyethylene alkyl phenyl ether phosphoric acid, N-acylamino acid salt, sodium stearate, potassium palmitate, sodium cetyl sulfate, sodium lauryl sulfate, triethanolamine palmitate, sodium polyoxyethylene lauryl phosphate, sodium acyl glutamate, and surfactin; cationic surfactants such as benzalkonium chloride, benzethonium chloride, stearyl trimethylammonium chloride, distearyl dimethylammonium chloride, and stearyl dimethylbenzyl ammonium chloride; amphoteric surfactants such as alkyldiaminoethylglycine hydrochloride, 2-alkyl-N-carboxymethyl-N-hydroxyethyl imidazolinium betaine, lauryl dimethyl amino acetic acid betaine, and lecithin; nonionic surfactants such as polyol fatty acid ester, glyceryl monostearate, lipophilic glyceryl monooleate, ethylene glycol monostearate, propylene glycol monostearate, sorbitan fatty acid ester, polyoxyethylene sorbitan fatty acid ester, polyoxyethylene fatty acid ester, polyoxyethylene alkyl ether, polyoxyethylene alkyl phenol ether, polyoxyethylene sorbitol fatty acid ester, N-acylamino acid ester, sucrose fatty acid ester, fatty acid alkylolamide, polyoxyethylenized sterol, polyoxyethylenized lanolin, and polyoxyethylene hard castor oil; fatty acid salts such as potassium laurate and potassium myristate; alkyl sulfuric acid ester salts such as sodium lauryl sulfate, triethanolamine lauryl sulfate, and ammonium lauryl sulfate; polyoxyethylene alkyl sulfates such as sodium laureth sulfate and triethanolamine laureth sulfate; acyl N-methyl amino acid salts such as sodium cocoyl methyl taurine, potassium cocoyl methyl taurine, sodium lauroyl methyl taurine, sodium myristoyl methyl taurine, sodium lauroyl methyl alanine, sodium lauroyl sarcosine, triethanolamine lauroyl sarcosine, and methyl alanine sodium lauroyl glutamate; acylamino acid salts such as sodium cocoyl glutamate, triethanolamine cocoyl glutamate, sodium lauroyl glutamate, sodium myristoyl glutamate, sodium stearoyl glutamate, ditriethanolamine palmitoyl aspartate, and triethanolamine cocoyl alanine; polyoxyethylene alkyl ether acetates such as sodium laureth acetate; succinic acid ester salts such as sodium lauroyl monoethanolamide succinate; fatty acid alkanolamide ether carboxylates; acyl lactates; polyoxyethylene fatty amine sulfates; fatty acid alkanolamide sulfates; fatty acid glyceride sulfates such as hard coconut oil fatty acid glycerin sodium sulfate; alkyl benzene polyoxyethylene sulfate; olefin sulfonates such as sodium α-olefin sulfonate; alkyl sulfosuccinates such as lauryl disodium sulfosuccinate, and sodium dioctyl sulfosuccinate; alkyl ether sulfosuccinates such as laureth disodium sulfosuccinate, monolauroyl monoethanolamide polyoxyethylene sulfosuccinic acid sodium, and lauryl polypropylene glycol sulfosuccinic acid sodium; alkyl benzene sulfonates such as sodium tetradecyl benzene sulfonate and triethanolamine tetradecyl benzene sulfonate; alkyl naphthalene sulfonates; alkane sulfonates; α-sulfo fatty acid methyl ester salt; acyl isethionates; alkyl glycidyl ether sulfonates; alkyl sulfoacetates; alkyl ether phosphoric acid ester salts such as sodium laureth phosphate, sodium dilaureth phosphate, sodium trilaureth phosphate, and sodium monooreth phosphate; alkyl phosphoric acid ester salts such as potassium lauryl phosphate; sodium caseinate; alkyl aryl ether phosphates; fatty acid amide ether phosphate; phospholipids such as phosphatidyl glycerol, phosphatidyl inositol, and phosphatidic acid; anionic surfactants including silicone-based anionic surfactants such as carboxylic acid-modified silicone, phosphoric acid-modified silicone, and sulfuric-acid modified silicone; polyoxyethylene alkyl ethers having various polyoxyethylene addition numbers such as laureths (polyoxyethylene lauryl ethers), ceteths (polyoxyethylene cetyl ethers), steareths (polyoxyethylene stearyl ethers), beheneths (polyoxyethylene behenyl ethers), isosteareths (polyoxyethylene isostearyl ethers), and octyldodeceths (polyoxyethylene octyl dodecyl ethers); polyoxyethylene alkyl phenyl ethers; castor oil and hard castor oil derivatives such as polyoxyethylene hard castor oil, polyoxyethylene castor oil, polyoxyethylene hard castor oil monoisostearate, polyoxyethylene hard castor oil triisostearate, polyoxyethylene hard castor oil monopyroglutamic acid monoisostearic acid diester, and polyoxyethylene hard castor oil maleic acid; polyoxyethylene phytosterol; polyoxyethylene cholesterol; polyoxyethylene cholestanol; polyoxyethylene lanolin; polyoxyethylene reduced lanolin; polyoxyethylene-polyoxypropylene alkyl ethers such as polyoxyethylene-polyoxypropylene cetyl ether, polyoxyethylene-polyoxypropylene 2-decyl tetradecyl ether, polyoxyethylene-polyoxypropylene monobutyl ether, polyoxyethylene-polyoxypropylene hydrogenated lanolin, and polyoxyethylene-polyoxypropylene glycerin ether; polyoxyethylene-polyoxypropylene glycol; (poly)glycerin polyoxypropylene glycols such as PPG-9 diglyceryl; glycerin fatty acid partial esters such as glyceryl stearate, glyceryl isostearate, glyceryl palmitate, glyceryl myristate, glyceryl oleate, coconut oil fatty acid glyceryl ester, mono cottonseed oil fatty acid glycerin ester, glyceryl monoerucate, glyceryl sesquioleate, α,α'-oleic acid pyroglutamic acid glycerin ester, and monostearic acid glycerin maleic acid ester; polyglycerol fatty acid esters such as polyglyceryl-2 stearate, polyglyceryl-3 stearate, polyglyceryl-4 stearate, polyglyceryl-5 stearate, polyglyceryl-6 stearate, polyglyceryl-8 stearate, and polyglyceryl-10 stearate, polyglyceryl-6 distearates, polyglyceryl-10 distearate, polyglyceryl-2 tristearate, polyglyceryl-10 decastearate, polyglyceryl-2 isostearate, polyglyceryl-3 isostearate, polyglyceryl-4 isostearate, polyglyceryl-5 isostearate, polyglyceryl-6 isostearate, polyglyceryl-8 isostearate, polyglyceryl-10 isostearate, polyglyceryl-2 diisostearate (diglyceryl diisostearate), polyglyceryl-3 diisostearate, polyglyceryl-10 diisostearate, polyglyceryl-2 triisostearate, polyglyceryl-2 tetraisostearate, polyglyceryl-10 decaisostearate, polyglyceryl-2 oleate, polyglyceryl-3 oleate, polyglyceryl-4 oleate, polyglyceryl-5 oleate, polyglyceryl-6 oleate, polyglyceryl-8 oleate, polyglyceryl-10 oleate, polyglyceryl-6 dioleate, polyglyceryl-2 trioleate, and polyglyceryl-10 decaoleate; ethylene glycol mono fatty acid esters such as ethylene glycol monostearate; propylene glycol mono fatty acid esters such as propylene glycol monostearate; pentaerythritol partial fatty acid esters; sorbitol partial fatty acid esters; maltitol partial fatty acid esters; maltitol ethers; sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, pent-2-ethylhexyl acid diglycerol sorbitan, and tetra-2-ethylhexyl acid diglycerol sorbitan; and sugar derivative partial esters such as sucrose fatty acid ester, methyl glucoside fatty acid ester, and trehalose undecylenate; alkyl glucosides such as caprylyl glucoside; alkyl polyglycosides; lanolin alcohols; reduced lanolins; polyoxyethylene fatty acid mono- and diesters such as polyoxyethylene distearate, polyethylene glycol diisostearate, polyoxyethylene monooleate, and polyoxyethylene dioleate; polyoxyethylene-propylene glycol fatty acid esters; polyoxyethylene glycerin fatty acid esters including polyoxyethylene monooleates such as polyoxyethylene glycerin monostearate, polyoxyethylene glycerin monoisostearate, and polyoxyethylene glycerin triisostearate; polyoxyethylene sorbitan fatty acid esters such as polyoxyethylene sorbitan monooleate, polyoxyethylene sorbitan monostearate, polyoxyethylene sorbitan monooleate, and polyoxyethylene sorbitan tetraoleate; polyoxyethylene sorbitol fatty acid esters such as polyoxyethylene sorbitol monolaurate, polyoxyethylene sorbitol monooleate, polyoxyethylene sorbitol pentaoleate, and polyoxyethylene sorbitol monostearate; polyoxyethylene methyl glucoside fatty acid esters; polyoxyethylene alkyl ether fatty acid esters; polyoxyethylene animal and plant fats and oils such as polyoxyethylene sorbitol beeswax; alkyl glyceryl ethers such as isostearyl glyceryl ether, chimyl alcohol, selachyl alcohol, and batyl alcohol; polyhydric alcohol alkyl ethers; polyoxyethylene alkyl amines; tetrapolyoxyethylene-tetrapolyoxypropylene-ethylenediamine condensates; natural-based surfactants such as saponin and sophorolipid; polyoxyethylene fatty acid amides; fatty acid alkanolamides such as coconut oil fatty acid monoethanolamide (cocamide MEA), coconut oil fatty acid diethanolamide (cocamide DEA), lauric acid monoethanolamide (lauramide MEA), lauric acid diethanolamide (lauramide DEA), lauric acid monoisopropanolamide (lauramide MIPA), palmitic acid monoethanolamide (palmitamide MEA), palmitic acid diethanolamide (palmitamide DEA), and coconut oil fatty acid methyl ethanolamide (cocamide methyl MEA); alkyl dimethyl amine oxides such as lauramine oxide, cocamine oxide, stearamine oxide, and behenamine oxide; alkyl ethoxy dimethyl amine oxides; polyoxyethylene alkyl mercaptan; nonionic surfactants including silicone-based nonionic surfactants such as polyether-modified silicones such as dimethicone copolyol, a polysiloxane-oxyalkylene copolymer, polyglycerin-modified silicone, and sugar-modified silicone; and as cationic surfactants, alkyl trimethyl ammonium chlorides such as behentrimonium chloride, steartrimonium chloride, setrimonium chloride, and lauryltrimonium chloride; alkyl trimethylammonium bromides such as stearyltrimonium bromide; dialkyl dimethyl ammonium chlorides such as distearyldimonium chloride and dicocodimonium chloride; fatty acid amidoamines such as stearamidopropyl dimethylamine and stearamidoethyl diethylamine and salts thereof; alkyl ether amine such as stearoxypropyl dimethyl amine and salts or quaternary salts thereof; fatty acid amide type quaternary ammonium salts such as ethyl sulfuric acid long-chain branched fatty acid (12 to 31) amino propyl ethyl dimethyl ammonium and ethyl sulfuric acid lanolin fatty acid amino propyl ethyl dimethyl ammonium; polyoxyethylene alkyl amines and salts or quaternary salts thereof; alkyl amine salts; fatty acid amide guanidinium salts; alkyl ether ammonium salts; alkyl trialkylene glycol ammonium salts; benzalkonium salts; benzethonium salts; pyridinium salts such as cetylpyridinium chloride; imidazolinium salts; alkyl isoquinolinium salts; dialkyl moriphonium salts; polyamine fatty acid derivatives; silicone-based cationic surfactants such as amino-modified silicones such as aminopropyl dimethicone and amodimethicone, cation-modified silicone, cation-modified and polyether-modified silicones, and amino-modified and polyether-modified silicones; N-alkyl-N,N-dimethyl amino acid betaine such as lauryl betaine (lauryl dimethyl amino acetic acid betaine); fatty acid amidoalkyl-N,N-dimethyl amino acid betaine such as cocamidopropyl betaine and lauramidopropyl betaine; imidazoline type betaines such as sodium cocoamphoacetate and sodium lauroamphoacetate; alkyl sulfobetaines such as alkyl dimethyl taurine; sulfuric acid type betaines such as alkyl dimethyl amino ethanol sulfuric acid ester; phosphoric acid type betaines such as alkyl dimethyl amino ethanol phosphoric acid ester; sphingophospholipids such as phosphatidyl choline, phosphatidyl ethanolamine, phosphatidyl serine, and sphingomyelin, phospholipids such as lysolecithin, hydrogenated soybean phospholipid, partially hydrogenated soybean phospholipid, hydrogenated egg yolk phospholipid, partially hydrogenated egg yolk phospholipid, and hydroxide lecithin; amphoteric surfactants such as silicone-based amphoteric surfactants; polyvinyl alcohol, sodium alginate, starch derivatives, tragacanth gum, and an acrylic acid-methacrylic acid alkyl copolymer; and polymer surfactants such as various silicone-based surfactants.

Examples of the polymerizing-thickening-gelling agent include guar gum, locust bean gum, queens seed, carrageenan, galactan, gum arabic, tara gum, tamarind, furcellaran, karaya gum, hibiscus, chara gum, tragacanth, pectin, pectic acid and salts thereof such as a sodium salt thereof, alginic acid acid and salts thereof such as a sodium salt thereof, and mannan; starches of rice, corn, potato, and wheat; xanthan gum, dextran, succinoglucan, curdlan, hyaluronic acid and salts thereof, xanthan gum, pullulan, gellan gum, chitin, chitosan, agar, brown alga extract, chondroitin sulfate, casein, collagen, gelatin, and albumin; methyl cellulose, ethyl cellulose, hydroxyethyl cellulose, hydroxypropyl cellulose, carboxymethyl cellulose and salts thereof such as a sodium salt thereof, and celluloses such as methyl hydroxypropyl cellulose, sodium cellulose sulfate, dialkyl dimethyl ammonium cellulose sulfate, crystalline cellulose, and cellulose powder, and derivatives thereof; starch-based polymers such as soluble starch, carboxymethyl starch, methyl hydroxypropyl starch, and methyl starch, and starch derivatives such as starch hydroxypropyltrimonium chloride and aluminum corn starch octenylsuccinate; alginic acid derivatives such as sodium alginate and alginic acid propylene glycol ester; polyvinyl polyvinylpyrrolidone (PVP), polyvinyl alcohol (PVA), a vinylpyrrolidone-vinyl alcohol copolymer, and polyvinyl methyl ether; polyethylene glycol, polypropylene glycol, and a polyoxyethylene-polyoxypropylene copolymer; amphoteric methacrylic acid ester copolymers such as a (methacryloyloxyethyl carboxybetaine/alkyl methacrylate) copolymer and an (acrylate/stearyl acrylate/ethylamine oxide methacrylate) copolymer; a (dimethicone/vinyl dimethicone) cross polymer, an (alkyl acrylate/diacetone acrylamide) copolymer, and an (alkyl acrylate/diacetone acrylamide) copolymer AMP; a polyvinyl acetate partial saponification product and a maleic acid copolymer; a vinylpyrrolidone-dialkyl aminoalkyl methacrylate copolymer; acrylic resin alkanolamine; polyester and water-dispersible polyester; polyacrylamide; plyacrylic acid ester copolymers such as ethyl polyacrylate, a carboxyvinyl polymer, polyacrylic acid and salts thereof such as a sodium salt thereof, and an acrylic acid-methacrylic acid ester copolymer; acrylic acid-alkyl methacrylate copolymers; cationized celluloses such as polyquaternium-10, diallyl dimethyl ammonium chloride-acrylamide copolymers such as polyquaternium-7, acrylic acid-diallyl dimethyl ammonium chloride copolymers such as polyquaternium-22, acrylic acid-diallyl dimethyl ammonium chloride-acryl amide copolymer such as polyquaternium-39, an acrylic acid-cationized methacrylic acid ester copolymer, an acrylic acid-cationized methacrylic acid amide copolymer, an acrylic acid-methyl acrylate-methacrylamide propyl trimethyl ammonium chloride copolymer such as polyquaternium-47, and a methacrylate chloride choline ester polymer; cationized polysaccharides such as cationized oligosaccharide, cationized dextran, and guar hydroxypropyl trimonium chloride; polyethyleneimine; cationic polymers; copolymers with a polymer of 2-methacryloyloxyethyl phosphorylcholine such as polyquaternium-51 and an butyl methacrylate copolymer; polymer emulsions such as an acrylic resin emulsion, polyacrylic acid ethyl emulsion, an polyacrylic alkyl ester emulsion, a polyvinyl acetate resin emulsion, natural rubber latex, and synthetic latex; nitrocellulose; polyurethanes and various copolymers; various silicones; various silicone-based copolymers such as an acryl-silicone graft copolymer; various fluorine-based polymers; 12-hydroxystearic acid and salts thereof; dextrin fatty acid esters such as dextrin palmitic acid ester, dextrin stearic acid ester, and dextrin 2-ethyl hexanoic acid palmitic acid ester; dextrin fatty acid esters such as dextrin myristate; organically modified clay minerals such as silicic anhydride, fumed silica (ultrafine particle silicic anhydride), aluminum magnesium silicate, sodium magnesium silicate, a metal soap, dialkyl phosphoric acid metal salt, bentonite, hectorite, dimethyl benzyldodecyl ammonium montmorillonite clay, and dimethyl dioctadecyl ammonium montmorillonite clay, sucrose fatty acid esters such as sucrose palmitic acid ester and sucrose stearic acid ester, amino acid derivatives such as N-lauroyl-L-glutamic acid and α, γ-di-n- butylamine, benzylidene derivatives of sorbitol such as monobenzylidene sorbitol and dibenzylidene sorbitol, and fructooligosaccharide fatty acid ester.

Examples of the solvent and the propellant include monohydric alcohols such as methanol, ethanol, propanol, benzyl alcohol, phenethyl alcohol, isopropyl alcohol, isobutyl alcohol, hexyl alcohol, 2-ethyl hexanol, cyclohexanol, octyl alcohol, butanol, and pentanol; ketones such as acetone and methyl ethyl ketone; lower alcohols such as ethanol, 2-propanol (isopropyl alcohol), butanol, and isobutyl alcohol; glycols such as propylene glycol, 1,3-butylene glycol, diethylene glycol, dipropylene glycol, and isopentyldiol; glycol ethers such as diethylene glycol monoethyl ether (ethoxydiglycol), ethylene glycol monoethyl ether, ethylene glycol monobutyl ether, triethylene glycol monoethyl ether, diethylene glycol diethyl ether, diethylene glycol dibutyl ether, propylene glycol monoethyl ether, and dipropylene glycol monoethyl ether; glycol ether esters such as ethylene glycol monoethyl ether acetate, diethylene glycol monoethyl ether acetate, and propylene glycol monomethyl ether acetate; glycol esters such as diethoxyethyl succinate and ethylene glycol disuccinate; benzyl alcohol, benzyloxyethanol, propylene carbonate, dialkyl carbonate, acetone, ethyl acetate, and N-methyl pyrrolidone; toluene; fluorocarbon and next generation freon; LPG, dimethyl ether, carbon dioxide, cellosolve, dioxane, dimethyl formamide, dimethyl sulfoxide, and crotamiton.

Examples of the antioxidant, the reductant, and the oxidant include tocopherol (vitamin E) and tocopherol derivatives such as tocopherol acetate; BHT and BHA; gallic acid derivatives such as propyl gallate; vitamin C (ascorbic acid) and/or derivatives thereof; erythorbic acid and derivatives thereof; sulfites such as sodium sulfite; bisulfite such as sodium bisulfite; thiosulfates such as sodium thiosulfate; meta hydrogensulfite; thiotaurine and hypotaurine; thioglycerol, thiourea, thioglycolic acid, cysteine hydrochloride, thioglycolic acid, cysteine, and cysteamine; hydrogen peroxide water, ammonium persulfate, sodium bromate, percarbonate, nordihydroguaiaretic acid, a guaiac resin, butyl hydroxyanisole, dibutyl hydroxytoluene (BHT), and 2,2'-methylenebis(4-methyl-6-t-butyl)phenol.

Examples of the preservative, the antibacterial agent, and the chelating agent include hydroxybenzoic acids such as methylparaben, ethylparaben, propylparaben, and butylparaben and salts thereof or esters thereof; salicylic acid: sodium benzoate; phenoxyethanol; 1,2-diols such as 1,2-pentanediol and 1,2-hexanediol; isothiazolinone derivatives such as methyl chloroisothiazolinone and methyl isothiazolinone; imidazolinium urea; dehydroacetic acid and salts thereof; phenols; halogenated bisphenols such as triclosan; acid amide, a quaternary ammonium salt; trichlorocarbanilide, zinc pyrithione, benzalkonium chloride, benzethonium chloride, sorbic acid, chlorhexidine, chlorhexidine gluconate, halocarban, hexachlorophene, hinokitiol; phenol, other phenols such as isopropyl phenol, cresol, thymol, parachlorophenol, phenylphenol, sodium phenylphenol; phenyl ethyl alcohol, photosensitizers, antibacterial zeolite, silver ion; edetates (ethylene diamine tetra-acetic acid salt) such as EDTA, EDTA2Na, EDTA3Na, and EDTA4Na; hydroxyethyl ethylenediamine triacetates such as HEDTA3Na; pentetate (diethylenetriamine pentaacetate); phytic acid; phosphonic acids such as etidronic acid, and salts thereof such as a sodium salt thereof; sodium oxalate, polyamino acids such as polyaspartic acid and polyglutamic acid; sodium polyphosphate, sodium metaphosphate, phosphoric acid; sodium citrate, citric acid, alanine, dihydroxyethylglycine, gluconate, ascorbic acid, succinic acid, tartaric acid, pyrophosphate, and hexametaphosphate.

Examples of the pH-adjusting agent, the acid, and alkali include alkali metal hydroxides and alkaline earth metal hydroxides such as sodium hydroxide, potassium hydroxide, calcium hydroxide, magnesium hydroxide, 2-amino-2-methyl-1,3-propanediol, 2-amino-2-hydroxymethyl-1,3-propanediol, arginine, guanidine carbonate, ammonium carbonate, ammonia, ammonia water, triethanolamine, dimethyl amine, diethylamine, trimethylamine, triethylamine, triisopropanolamine, trisodium phosphate, disodium hydrogen phosphate, dipotassium hydrogen phosphate, monoethanolamine, diethanolamine, isopropanol amine, diisopropanolamine, and polyethanolamine, alkalis such as primary, secondary, or tertiary alkyl amines, or primary, secondary, or tertiary alkanolamine, acids such as citric acid, tartaric acid, lactic acid, glycolic acid, hydrochloric acid, nitric acid, citric acid, sodium citrate, sodium lactate, succinic acid, acetic acid, sodium acetate, fumaric acid, sulfuric acid, malic acid, and phosphoric acid, polymers exhibiting acidity or alkalinity, such as alginic acid, polyglutamic acid, polyaspartic acid, a starch-acrylic acid graft polymer, polyacrylic acid, a polyvinyl acetate-crotonic acid copolymer, a vinyl acetate-(meth) acrylic acid copolymer, a vinyl acetate-crotonic acid copolymer, polyvinyl sulfonic acid, polyitaconic acid, a styrene-maleic anhydride copolymer, and an acrylamide-acrylic acid copolymer.

Examples of the powder and the inorganic salt include inorganic powders having various sizes and shapes, such as mica, talc, kaolin, sericite, montmorillonite, kaolinite, mica, white mica, phlogopite, synthetic mica, lepidolite, biotite, vermiculite, magnesium carbonate, calcium carbonate, aluminum silicate, barium silicate, calcium silicate, magnesium silicate, strontium silicate, metal tungstate, magnesium, zeolite, barium sulfate, calcined calcium sulfate, calcium phosphate, fluorine apatite, hydroxyapatite, a ceramic powder, bentonite, smectite, clay, mud, a metal soap (for example, zinc myristate, calcium palmitate, and aluminum stearate), calcium carbonate, red oxide, yellow iron oxide, black iron oxide, ultramarine blue, prussian blue, carbon black, titanium oxide, fine or ultrafine particles of titanium oxide, zinc oxide, fine and ultrafine particles of zinc oxide, alumina, silica, fumed silica (ultrafine particle silicic anhydride), mica titanium, argentine, boron nitride, a photochromic pigment, synthetic fluorine phlogopite, a fine particle composite powder, gold, and aluminum, and inorganic powders such as powders hydrophobized or hydrophilizied by treating these with various surface treatment agents such as silicones such as hydrogen silicone and cyclic hydrogen silicone, or other silanes or a titanate coupling agent; starch, cellulose, a nylon powder, a polyethylene powder, polymethyl methacrylate powder, polystyrene powder, a copolymer resin powder of styrene and acrylic acid, a polyester powder, a benzoguanamine resin powder, a polyethylene terephthalate-polymethyl methacrylate laminated powder, a polyethylene terephthalate-aluminum-epoxy laminated powder, organic powders and surface treatment powders having various sizes and shapes such as a urethane powder, a silicone powder, a teflon (registered trademark) powder, and an organic-inorganic composite powder; sodium chloride-containing salts such as salt, crude salt, rock salt, sea salt, and natural salt; potassium chloride, aluminum chloride, calcium chloride, magnesium chloride, bittern, zinc chloride, and ammonium chloride; sodium sulfate, aluminum sulfate, aluminum potassium sulfate (alum), aluminum ammonium sulfate, barium sulfate, calcium sulfate, potassium sulfate, magnesium sulfate, zinc sulfate, iron sulfate, and copper sulfate; sodium phosphates such as monosodium, disodium, trisodium phosphates, potassium phosphates, calcium phosphates, magnesium phosphate, magnesium oxide, lithia mica, silicic acid, aluminum magnesium silicate, vermiculite, higilite, a ceramic powder, dibasic calcium phosphate, aluminum hydroxide, and boron nitride; organic powders such as a polyamide powder, a polyester powder, a polyethylene powder, a polypropylene powder, a polystyrene powder, a polyurethane, a benzoguanamine powder, a polymethyl benzoguanamine powder, a tetrafluoroethylene powder, a polymethyl methacrylate powder, a silk powder, a nylon powder, 12 nylon, 6 nylon, a styrene-acrylic acid copolymer, a divinyl benzene-styrene copolymer, a vinyl resin, a urea resin, a phenol resin, a fluorine resin, a silicon resin, an acrylic resin, a melamine resin, an epoxy resin, a polycarbonate resin, a microcrystalline fiber powder, and lauroyl lysine; colored pigments including inorganic red pigments such as iron oxide, iron hydroxide, and titanium iron, inorganic brown pigments such as γ-iron oxide, inorganic yellow pigments such as yellow iron oxide and ocher, inorganic black pigments such as black iron oxide and carbon black, inorganic purple pigments such as manganese violet and cobalt violet, inorganic green pigments such as chromium hydroxide, chromium oxide, cobalt oxide, and cobalt titanate, inorganic blue pigments such as prussian blue and ultramarine blue, a powder obtained by laking a tar-based coloring matter, a powder obtained by laking natural coloring matters, and a composite powder obtained by compositing these powders; pearl pigments such as titanium oxide-coated mica, titanium oxide-coated mica, bismuth oxychloride, titanium oxide-coated bismuth oxychloride, titanium oxide-coated talc, argentine, and titanium oxide-coated colored mica; metal powder pigments such as an aluminum powder, a copper powder, and a stainless steel powder; tar coloring matters such as Red Nos. 3, 104, 106, 201, 202, 204, 205, 220, 226, 227, 228, 230, 401, and 505, Yellow Nos. 4, 5, 202, 203, 204, and 401, Blue Nos. 1, 2, 201, and 404, Green Nos. 3, 201, 204, and 205, and Orange Nos. 201, 203, 204, 206, and 207; a powder selected from natural coloring matters such as carmine acid, laccaic acid, carthamin, brazilin, and crocin, and powders obtained by forming a composite compound or performing a surface treatment on these powders with oil, silicone, or a fluorine compound, montmorillonite, silicic anhydride, gypsum, diatomaceous earth, calcium carbonate, hydrotalcite, talc, glass, kaolin, a metal soap, aerosil, bismuth oxychloride, argentine, zinc white, and titanium dioxide.

Examples of the ultraviolet absorbent include benzoic acid-based ultraviolet absorbents such as para-aminobenzoic acid, para-aminobenzoic acid mono glycerin ester, N,N-dipropoxy para-aminobenzoic acid ethyl ester, N,N-diethoxy para-aminobenzoic acid ethyl ester, N,N-dimethyl para-aminobenzoic acid ethyl ester, N,N-dimethyl-para-aminobenzoic acid butyl ester, and N,N-dimethyl para-aminobenzoic acid ethyl ester; anthranilic acid-based ultraviolet absorbents such as homomenthyl-N-acetyl anthranilate; salicylic acid-based ultraviolet absorbents such as salicylic acid and sodium salts thereof, amyl salicylate, menthyl salicylate, homomenthyl salicylate, octyl salicylate, phenyl salicylate, and benzyl salicylate, p-isopropanol phenyl salicylate; cinnamic acid-based ultraviolet absorbents such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxycinnamate, isopropyl-p-methoxycinnamate, isoamyl-p-methoxycinnamate, 2-ethylhexyl p-methoxycinnamate (octyl para-methoxycinnamate), 2-ethoxyethyl-p-methoxycinnamate (cinoxate), cyclohexyl-p-methoxycinnamate, ethyl-α-cyano-β-phenyl cinnamate, 2-ethylhexyl α-cyano-β-phenyl cinnamate (octocrylene), glyceryl mono-2-ethylhexanoyl- di-para-methoxycinnamate, and ferulic acid, and derivatives thereof; benzophenone-based ultraviolet absorbents such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone (oxybenzone-3), 2-hydroxy-4-methoxy-4'-methyl benzophenone, 2-hydroxy-4-methoxybenzophenone-5-sulfonate,4-phenyl benzophenone, 2-ethylhexyl-4'-phenyl-benzophenone-2-carboxylate, 2-hydroxy -4-n-octoxybenzophenone, and 4-hydroxy-3-carboxybenzophenone; 3-(4'-methylbenzylidene)-d,l-camphor, 3-benzylidene-d,l-camphor; 2-phenyl-5-methyl benzoxazole; 2,2'-hydroxy-5-methyl phenyl benzotriazole; 2-(2'-hydroxy-5'-t-octylphenyl)benzotriazole; 2-(2'-hydroxy-5'-methylphenyl benzotriazole; dibenzaladine; dianisoyl methane; 5-(3,3-dimethyl-2-norbornylidene)-3-pentan-2-one; and dibenzoylmethane derivatives such as 4-t-butyl methoxydibenzoylmethane; octyl triazone; urocanic acid derivatives such as urocanic acid and ethyl urocanate; 2-(2'-hydroxy-5'-methylphenyl)benzotriazole, 1-(3,4-dimethoxyphenyl)-4,4-dimethyl-1,3-pentanedione, hydantoin derivatives such as 2-ethylhexyl dimethoxybenzylidene dioxoimidazolidine propionate, phenylbenzimidazole sulfonic acid, terephthalylidene dicamphor sulfonic acid, drometrizole trisiloxane, methyl anthranilate, rutin and derivatives thereof, and oriyzanol and derivatives thereof.

Examples of the whitening agent include hydroquinone glycosides such as arbutin and α-arbutin, and esters thereof; ascorbic acid phosphoric acid ester salts such as ascorbic acid, an ascorbic acid phosphoric acid ester sodium salt, and an ascorbic acid phosphoric acid ester magnesium salt, ascorbic acid fatty acid ester such as ascorbic acid tetra isopalmitic acid ester, ascorbic acid alkyl ether such as ascorbic acid ethyl ether, ascorbic acid glucoside such as ascorbic acid 2-glucoside and fatty acid esters thereof, ascorbic acid derivatives such as ascorbic acid sulfuric acid ester and tocopheryl ascorbyl phosphate; kojic acid, ellagic acid, tranexamic acid and derivatives thereof, ferulic acid and derivatives thereof, placenta extract, glutathione, orizanol, butyl resorcinol, oil-soluble chamomilla extract, oil-soluble licorice extract, Chinese tamarisk extract, saxifrage extract, glabridin, glabrene, liquiritin, isoliquiritin, and licorice extract containing these, placenta extract, carotenoids and animal and plant extracts containing these, neoagarobiose, agarose oligosaccharide, asparagus extract, bistort extract, pea extract, rose fruit extract, scutellaria root extract, ononis spinosa extract, seaweed extract, raspberry extract, sophora root extract, mucuna birdwoodiana extract, gokahi extract, vegetable oil containing linoleic acid, saisin extract, hawthorn extract, cassia nomame extract, white lily extract, peony extract, inula flower extract, mulberry bark extract, soybean extract, tea extract, angelica extract, molasses extract, white lotus extract, beech extract, grape seed extract, flor de manita extract, hop extract, rosa rugosa extract, mokka extract, saxifrage extract, coix seed extract, and Momordica grosvenori extract, and extracts of plants such as asparagus, madder, red grape, mallotus, akebi, cannabis, morning glory, adzuki bean, gambir, sweet hydrangea, pentaphyllum, Japanese knotweed, fig tree, ginkgo, ylang-ylang, prunella, Japanese apricot, bearberry, satsuma mandarin, siberian ginseng, sicklepod, sophora, peas, plantain, okra, ragweed, juglans, golden lace, strawberry, kaki, ground ivy, polygonum, cashew, valerian, snake gourd, karin, guarana, bellflower, chrysanthemum, catalpa, sorrel, gymnema sylvestre, agrimonia, guava, wolfberry, arrowroot, camphor tree, chestnut, mucuna birdwoodiana, bay, cinnamon, raspberry, pepper, coffee, figwort, colombo, sasanqua, Japanese pepper, saffron, cherry tree, pomegranate, sophorae subprostrata radix, chamaecrista nomame, aster, calamus, watermelon, stevia, plum, english ivy, european pear, yarrow, juniperus communis, horseradish, sweet flag, water dropwort, senega, senna, rhubarb, bitter orange, tamarind, aralia elata, dandelion, chicory, clove, schisandra chinensis, umbrella polypore sclerotium, evening primrose, centella asiatica, dayflower, tetragonia tetragonoides, juglans regia, wax gourd, hardy rubber tree, sunset hibiscus, shepherd's purse, summer orange, nandina domestica thumb, picrasma wood, siberian yarrow, pineapple, hibiscus, papaya, basil, lotus, barley, blackberry lily, peanut, isodon japonicus, water caltrops, pistachio, cypress, agaricus blazei murill, angelicae dahurica, loquat, coltsfoot, Japanese sumac, thoroughwort, blueberry, saposhnikovia, ground cherry, magnolia, flowering quince, rosa maikwai, ephedra, mango, ganoderma lucidum, bupleurum, lythrum anceps, Japanese hornwort, mimosa, melilot, melon, lily magnolia, momordica-grosvenorii, Jews mallow, bean sprouts, bitter cardamon, leonurus, cornflower, palm, ulmus pumila, mistletoe, water pepper, pokeweed, bayberry, daphniphyllum macropodum, wormwood, rye, orchid, longan, apple, litchi, and weeoing forsythia.

Examples of the vitamins and derivatives thereof include vitamin A such as retinol, retinol acetate, retinol palmitate; vitamin B such as thiamine hydrochloride, thiamine sulfate, riboflavin, riboflavin acetate, riboflavin butyric acid ester, pyridoxine hydrochloride, pyridoxine dioctanoate, pyridoxine palmitate, flavin adenine dinucleotide, cyanocobalamin, folic acids, nicotinic acids such as nicotinic acid amide-benzyl nicotinate, and colines; vitamin C such as ascorbic acid and salts of sodium and the like thereof; vitamin D; vitamin E such as α, β, γ, and δ-tocopherols; other vitamins such as pantothenic acid and biotin; ascorbic acid phosphoric acid ester salts such as an ascorbic acid phosphoric acid ester sodium salt and an ascorbic acid phosphoric acid ester magnesium salt, ascorbic acid fatty acid ester such as ascorbic acid tetraisopalmitic acid ester-stearic acid ascorbyl-palmitic acid ascorbyl-dipalmitic acid ascorbyl, ascorbic acid alkyl ethers such as ascorbic acid ethyl ether, ascorbic acid glucosides such as ascorbic acid 2-glucoside, fatty acid esters thereof, and ascorbic acid derivatives such as phosphoric acid tocopheryl ascorbyl; vitamin derivatives including tocopherol derivatives such as tocopherol nicotinate, tocopherol acetate, tocopherol linoleate, tocopherol ferulate, and tocopherol phosphoric acid ester, and tocotrienol.

Examples of the antiphlogistic agent, the antiinflammatory agent, the drug for hair growth, the blood circulation accelerator, the stimulant, the hormone, the antiwrinkle agent, the antiaging agent, the tightening agent, the cold sense agent, the warm sense agent, the wound healing accelerator, the irritation alleviating agent, the analgesic, and the cell activator include glycyrrhizic acid and derivatives thereof, glycyrrhetinic acid derivatives, salicylic acid derivatives, hinokitiol, guaiazulene, allantoin, indomethacin, zinc oxide, hydrocortisone acetate, prednisone, diphedoramine hydrochloride, and chlorpheniramine maleate; plant extracts such as peach leaf extract and mugwort extract; plant extracts or tinctures such as Japanese green gentian extract, pepper tincture, ginger tincture, ginger extract, and cantharis tincture; capsaicin, nonanoic acid vanillylamide, zingerone, ichthammol, tannic acid, borneol, cyclandelate, cinnarizine, tolazoline, acetylcholine, verapamil, cepharanthine, γ-orizanol, cepharanthine, vitamin E, and derivatives of tocopherol nicotinate·tocopherol acetate, γ-orizanol, nicotinic acid, derivatives of nicotinic acid such as nicotinic acid amide·nicotinic acid benzyl ester·inositol hexanicotinate, nicotine alcohol, allantoin, photosensitizers 301 and 401, carpronium chloride, pentadecanoic acid monoglyceride, flavanonol derivatives, stigmasterol or stigmastanol and glycosides thereof, and minoxidil; estradiol, estrone, ethinyl estradiol, cortisone, hydrocortisone, and prednisone; retinols, retinoic acids, and tocopheryl retinoic acid; derivatives such as lactic acid, glycolic acid, gluconic acid, fruit acid, salicylic acid, and glycosides thereof·esterification product, α- or β-hydroxy acids such as hydroxy capric acid, long-chain α-hydroxy fatty acid, and long-chain α-hydroxy fatty acid cholesteryl, and derivatives thereof; γ-amino butyric acid and γ-amino-β-hydroxybutyric acid; carnitine; creatine; ceramides and sphingosines; caffeine, xanthine, and derivatives thereof; antioxidants and active oxygen scavengers such as coenzyme Q10, carotene, lycopene, and astaxanthin; catechins; flavones such as quercetin; isoflavones; gallic acid and ester sugar derivatives; polyphenols such as tannin, sesamin, protoanthocyanidin, chlorogenic acid, and apple polyphenol; derivatives such as rutin and glycosides; derivatives such as hesperidin and glycosides; lignan glycoside; licorice extract-related substances such as glabridin, glabrene, liquiritin, and isoliquiritin; lactoferrin; shogaol and gingerol; fragrance substances and derivatives thereof such as menthol, camphor, and cedrol; capsaicin, vanillin, and derivatives thereof; insect repellents such as diethyltoluamide; and a complex of a physiologically active substance and cyclodextrin.

Examples of the plant, animal, and microbial extract include iris extract, ashitaba extract, thujopsis dolobrata extract, asparagus extract, avocado extract, sweet hydrangea leaf extract, almond extract, altea extract, arnica extract, aloe extract, apricot extract, apricot kernel extract, ginkgo extract, inchikow extract, fennel extract, turmeric extract, oolong tea extract, uva-ursi extract, rose fruit extract, echinacea leaf extract, isodon japonicus extract, scutellaria root extract, phellodendron bark extract, coptis japonica extract, barley extract, panax ginseng extract, hypericum extract, lamium album extract, ononis extract, Netherland mustard extract, orange extract, a dried seawater product, seaweed extract, persimmon leaf extract, pyracantha fortuneana fruit extract, hydrolyzed elastin, a hydrolyzed wheat powder, hydrolyzed silk, pueraria root extract, chamomilla extract, oil-soluble chamomilla extract, carrot extract, capillary artemisia extract, wild oat extract, hibiscus sabdariffa extract, licorice extract, oil-soluble licorice extract, kiwi extract, kiou apple extract, Jew's ear mushroom extract, cinchona bark extract, cucumber extract, paulownia tomentosa leaf extract, guanosine, guava extract, sophora root extract, gardenia extract oil, kuma bamboo grass extract, sophora flavescens extract, walnut extract oil, chestnut extract, grapefruit extract, clematis extract, black rice extract, brown sugar extract, black vinegar, chlorella extract, mulberry extract, gentian extract, geranium thunbergii extract, black tea extract, yeast extract, magnolia bark extract, coffee extract, burdock extract, rice extract, fermented rice extract, fermented rice bran extract, rice germ, comfrey extract, collagen, cowb extract, saisin extract, bupleurum root extract, umbilical cord extract liquid, saffron extract, salvia extract, saponaria officinalis extract, sasa extract, hawthorn extract, sansha extract, Japanese pepper extract, shiitake mushroom extract, rehmannia root extract, lithospermum root extract, perilla extract, Japanese linden extract, meadowsweet extract, jatoba extract, peony extract, ginger extract, calamus root extract, white birch extract, white Jew's ear mushroom extract, horsetail extract, stevia extract, a stevia-fermented product, Chinese tamarisk extract, English ivy extract, whitethorn extract, Sambucus nigra extract, yarrow extract, peppermint extract, sage extract, mallow extract, cnidium rhizome extract, Japanese green gentian extract, mulberry bark extract, rhubarb extract, soybean extract, jujube extract, thyme extract, dandelion extract, lichens extract, tea extract, clove extract, imperata extract, citrus unshiu peel extract, tea tree extract, Chinese blackberry extract, capsicum extract, Japanese angelica root extract, calendula officinalis extract, peach kernel extract, spruce extract, houttuynia extract, tomato extract, natto extract, carrot extract, garlic extract, eglantine extract, hibiscus extract, ophiopogon extract, lotus extract, parsley extract, birch extract, honey, hamamelis extract, parietaria extract, isodon japonicus extract, bisabolol, cypress extract, lactobacillus bifidus extract, loquat extract, coltsfoot extract, petasites japonicus extract, hoelen extract, butcher bloom extract, grape extract, grape seed extract, propolis, loofah extract, safflower extract, peppermint extract, linden extract, tree peony extract, hop extract, rosa rugosa extract, pine extract, horse chestnut extract, skunk cabbage extract, soapberry extract, melissa extract, mozuku extract, peach extract, cornflower extract, eucalyptus extract, saxifrage extract, yuzu extract, lily extract, coix seed extract, wormwood extract, lavender extract, green tea extract, egg shell membrane extract, apple extract, rooibos tea extract, litchi extract, lettuce extract, lemon extract, weeping forsythia extract, astragalus sinicus extract, rose extract, rosemary extract, roman chamomile extract, royal jelly extract, and burnet extract.

Examples of the antipruritic agent, the keratin releasing-dissolving agent, the antiperspirant, the cooling agent, the astringent agent, the enzyme, and the nucleic acid include diphenhydramine hydrochloride, chlorpheniramine maleate, camphor, and a substance-P inhibitor; salicylic acid, sulfur, resorcinol, selenium sulfide, and pyridoxine; chlorohydroxyaluminum, aluminum chloride, zinc oxide, and zinc para-phenol sulfonate; menthol and methyl salicylate; citric acid, tartaric acid, lactic acid, aluminum potassium sulfate, and tannic acid; superoxide dismutase, catalase, lysozyme chloride, lipase, papain, pancreatin, and protease; ribonucleic acid and salts thereof, deoxyribonucleic acid and salts thereof, and adenosine triphosphate disodium.

Examples of the fragrance include vegetable fragrances such as mustard oil, orange oil, pepper oil, jasmine oil, cedar oil, calamus oil, terpin oil, neroli oil, rose oil, eucalyptus oil, lime oil, lemon oil, Japanese mint oil, and rosemary oil; animal fragrances such as musk, civet, castoreum, and ambergris; hydrocarbon-based fragrances such as bromostyrol, pinene, and limonene; alcohol-based fragrances such as benzyl alcohol and 1-menthol; ester-based fragrances such as ethyl acetate and methyl salicylate; aldehyde-based fragrances such as benzaldehyde and salicylaldehyde; ketone-based fragrances such as camphor, muscone, musk ketone, and 1-menthone; ether-based fragrances such as safrole; phenol-based fragrances such as thymol; lactone-based fragrances; acid-based fragrances such as phenylacetic acid; nitrogen compound-based fragrances such as indole; and synthetic fragrances, natural fragrances, and various compound fragrances such as acetyl cedrene, amyl cinnamaldehyde, allylamyl glycolate, β-ionone, iso E super, isobutyl quinoline, iris oil, irone, ylang ylang oil, undecanal, undecenal, γ-undecalactone, estragole, oakmoss, opoponax resinoid, eugenol, aurantiol, galaxolide, carvacrol, L-carvone, cannon, carrot seed oil, clove oil, methyl cinnamate, geraniol, geranyl nitrile, isobornyl acetate, geranyl acetate, dimethyl benzyl carbinylacetate, styralyl acetate, cedryl acetate, terpinyl acetate, p-t-butylcyclohexyl acetate, vetiveryl acetate, benzyl acetate, linalyl acetate, isopentyl salicylate, benzyl salicylate, sandalwood oil, santalol, cyclamen aldehyde, cyclopentadecanolide, methyl dihydrojasmonate, dihydromyrcenol, jasmine absolute, jasmine lactone, cis-jasmone, citral, citronellol, citronellal, cinnamon bark oil, 1,8-cineole, cinnamaldehyde, styrax resinoid, cedarwood oil, cedrene, cedrol, celery seed oil, thyme oil, damascone, damascenone, tuberose absolute, decanal, decalactone, terpineol, γ-terpinene, triplal, nerol, nonanal, 2,6-nonadienol, nonalactone, patchouli alcohol, vanilla absolute, vanillin, basil oil, patchouli oil, hydroxycitronellal, α-pinene, piperitone, phenethyl alcohol, phenyl acetaldehyde, petitgrain oil, hexylcinnamaldehyde, cis-3-hexenol, Peru balsam, vetiver oil, vetiverol, peppermint oil, pepper oil, heliotropine, bergamot oil, benzyl benzoate, borneol, myrrh resinoid, methyl nonyl acetaldehyde, γ-methylionone, menthol, β-ionone, lavender oil, D-limonene, linalool, lyral, lilial, rose absolute, rose oxide, rose oil, and various essential oils.

Examples of the coloring matter, colorant, dye, and pigment include certified coloring matter such as Brown No. 201, Black No. 401, Purple Nos. 201 and 401, Blue Nos. 1, 2, 201, 202, 203, 204, 205, 403, and 404, Green Nos. 201, 202, 204, 205, 3, 401, and 402, Red Nos. 102, 104-1, 105-1, 106, 2, 201, 202, 203, 204, 205, 206, 207, 208, 213, 214, 215, 218, 219, 220, 221, 223, 225, 226, 227, 228, 230-1, 230-2, 231, 232, 3, 401, 404, 405, 501, 502, 503, 504, 505, and 506, Orange Nos. 201, 203, 204, 205, 206, 207, 401, 402, and 403, Yellow Nos. 201, 202-1, 202-2, 203, 204, 205, 4, 401, 402, 403-1, 404, 405, 406, 407, and 5; other acidic dyes such as Acid Red 14; basic dyes such as Arianor Sienna Brown, Arianor Madder Red, Arianor Steel Blue, and Arianor Straw Yellow; nitro dyes such as HC Yellow 2, HC Yellow 5, HCRed 3, 4-hydoxypropylamino-3-nitrophenol, N,N'-bis (2-hydroxyethyl)-2-nitro-p-phenylenediamine, HC Blue 2, and Basic Blue 26; disperse dyes; inorganic white pigments such as titanium dioxide and zinc oxide; inorganic red pigments such as iron oxide (red iron oxide) and iron titanate; inorganic brown pigments such as γ-iron oxide; inorganic yellow pigments such as yellow iron oxide and ocher, inorganic black pigments such as black iron oxide and lower titanium oxide; inorganic purple pigments such as mango violet and cobalt violet; inorganic green pigments such as chromium oxide, chromium hydroxide, and cobalt titanate; inorganic blue pigments such as ultramarine blue and prussian blue; pearl pigments such as titanium oxide coated mica, titanium oxide coated bismuth oxychloride, titanium oxide coated talc, colored titanium oxide coated mica, bismuth oxychloride, and argentine; metal powder pigments such as an aluminum powder, a copper powder, and gold; surface treatment inorganic and metal powder pigments; organic pigments including zirconium, barium, or aluminum lake such as Red Nos. 201, 202, 204, 205, 220, 226, 228, and 405, Orange Nos. 203, 204, 205, and 401, Blue No. 404, Red Nos. 3, 104, 106, 227, 230, 401, and 505, Orange No. 205, Yellow Nos. 4, 5, 202, and 203, Green No. 3, and Blue No. 1; surface treatment organic pigments; natural coloring matter and dyes such as anthraquinones such as astaxanthin and alizarin, naphthoquinones such as anthocyanidin, β-carotene, carthenal, capsanthin, chalcone, carthamin, quercetin, crocin, chlorophyll, curcumin, cochineal, and shikonin, bixin, flavones, betacyanidin, henna, hemoglobin, lycopene, riboflavin, and rutin; oxidation dye intermediates and couplers such as p-phenylenediamine, toluene-2,5-diamine, o-, m-, or p-aminophenol, m-phenylenediamine, 5-amino-2-methyl phenol, resorcinol, 1-naphthol, 2,6-diaminopyridine, and salts thereof; auto-oxidation type dyes such as indoline; and dihydroxyacetone.

Examples of the water include common water, purified water, and hard water, soft water, natural water, deep ocean water, electrolytic alkaline ion water, electrolytic acidic ion water, ion water, and cluster water.

Examples of the metal-containing compound include lithium compounds such as lithium oxide, butyl lithium, ethyl lithium, lithium bromide, lithium carbide, lithium iodide, lithium nitrate, lithium nitride, lithium sulfide, methyl lithium, lithium sulfate, lithium chloride, lithium hydride, propyl lithium, lithium carbonate, lithium fluoride, lithium niobate, lithium peroxide, lithium hydroxide, lithium molybdate, lithium perchlorate, lithium acetate, lithium formate, lithium tungstate, lithium hypochlorite, lithium acetate dihydrate, lithium iodide dihydrate, llithium hydrogensulfate, lithium acetate dihydrate, lithium bromide monohydrate, lithium sulfate monohydrate, dilithium carbonate, dilithium oxalate, dilithium sulfate, lithium chromate dihydrate, lithium hydroxide monohydrate, lithium citrate tetrahydrate, lithium perchlorate trihydrate, and lithium hexafluorophosphate; sodium compounds such as sodium amide, sodium oxide, sodium azide, sodium acetate, sodium acetylide, sodium bromide, sodium formate, sodium iodide, sodium lactate, sodium methoxide, sodium nitrate, sodium sulfate, sodium sulfide, sodium borate, metallic sodium, sodium bromate, sodium chloride, sodium hydride, sodium nitrite, sodium sulfite, sodium iodate, sodium phosphate, ecabet sodium, sodium butyrate, heparin sodium, sodium chlorate, sodium citrate, sodium cyanate, sodium cyanide, sodium fluoride, sodium oleate, sodium oxalate, sodium peroxide, sodium silicate, sodium stannate, sodium-t-butoxide, sodium carbonate, suramin sodium, sodium benzoate, sodium chlorite, sodium chromate, sodium hydroxide, sodium selenate, sodium selenide, sodium laurate, disodium inosylate (hereinafter, referred to as IMP), sodium alginate, sodium perborate, sodium persulfate, sodium phosphite, sodium rhodanide, disodium arsenate, sodium selenite, amfenac sodium, tolmetin sodium, sodium pyruvate, saccharin sodium, sodium aluminate, sodium gluconate, sodium polysulfide, sodium stearate, sodium valproate, sodium iopodate, sodium bicarbonate, disodium carbonate, sodium periodate, reviparin sodium, porfimer sodium, carumonam sodium, cefminox sodium, flomoxef sodium, cefalotin sodium, cefazolin sodium, latamoxef sodium, sodium glutamate, sodium molybdate, sodium percarbonate, sodium perchlorate, sodium salicylate, sodium thiosulfate, thiamylal sodium, triclofos sodium, sodium hydrogen carbonate, sodium dichromate, sodium dichromate, sodium fluoroborate, sodium sulfoxylate, disodium phosphate, beraprost sodium, cefotetan sodium, ceftezole sodium, faropenem sodium, trisodium citrate, cefapirin sodium, ampicillin sodium, cefoxitin sodium, cefsulodin sodium, cefuroxime sodium, dantrolene sodium, sodium antimonate, sodium dithionite, sodium propionate, sodium tungstate, sodium pyrobisulfite, trisodium phosphate, sodium triphosphate, dalteparin sodium, parnaparin sodium, danaparoid sodium, cefodizime sodium, carmellose sodium, sodium iothalamate, foscarnet sodium, sodium metabisulfite, cefotaxime sodium, sefpiramide sodium, sodium cyclamate, diclofenac sodium, sodium borohydride, sodium hypochlorite, sodium thiocyanate, thiopental sodium, ticarcillin sodium, fosfomycin sodium, sodium arsenate, dibasic sodium phosphinate, sodium hyaluronate, imitrodast sodium, bucladesine sodium, sodium boron hydroxide, sodium glucuronate, sodium aluminum sulfate, cefmetazole sodium, ceftizoxime sodium, cloxacillin sodium, sodium cyclamate, fluorescein sodium, liothyronine sodium, loxoprofen sodium, piperacillin sodium, sodium hydrosulfite, sodium hypophosphite, sodium naphthionate, sodium permanganate, sodium pyrophosphate, fluvastatin sodium, pravastatin sodium, sodium rabeprazole, sodium methacrylate, disodium 5'-IMP, amobarbital sodium, cefamandole sodium, cefoperazone sodium, sodium ferrocyanide, sodium hydrogensulfide, sodium metavanadate, sodium silicofluoride, sodium xanthate, sodium fluoroacetate, sodium lauryl sulfate, sodium cromoglicate, sodium picosulfate, sodium fluoroacetate, sodium metabisulfite, sodium taurocholate, cerivastatin sodium, sodium acetate trihydrate, ceftriaxone sodium, carbenicillin sodium, cefbuperazone sodium, sodium fumarate, levothyroxine sodium, sodium dehydroacetate, sodium hydrogen sulfite, sulbenicillin sodium, sodium borate decahydrate, sodium metavanadate, sodium metaperiodate, sodium cholate hydrate, sodium sulfate decahydrate, sodium dodecyl sulfate, secobarbital sodium, lobenzarit disodium, sodium deoxycholate, sodium nitroprusside, dicloxacillin sodium, phthalocyanine sodium, sodium thioglycolate, sodium sulfate, sodium dihydronaphthalenidyl, gold sodium thiomalate, 2-biphenylol sodium, sodium cholate n-hydrate, sodium stannate trihydrate, sodium sulfide nonahydrate, calcium disodium edetate, epoprostenol sodium, sodium ferrous citrate, anhydrous sodium carbonate, sodium sulfite heptahydrate, sodium silicoaluminate, caffeine sodium benzoate, disodium hydrogenarsenate, saccharin sodium dihydrate, dibasic sodium phosphate, sodium trichloroacetate, sodium tripolyphosphate, sodium peroxodisulfate, bromfenac sodium hydrate, sodium molybdate dihydrate, basic sodium phosphate, tribasic sodium phosphate, sodium trifluoroacetate, tetrasodium pyrophosphate, sodium chromate tetrahydrate, sodium succinate hexahydrate, sodium dihydrogen citrate, sodium stearyl fumarate, antimony sodium tartrate, sodium cyanoborohydride, sodium dihydrogen phosphate, sodium prasterone sulfate, sodium selenite pentahydrate, dipicrylamine sodium, sodium hexametaphosphate, sodium perborate tetrahydrate, sodium tetrahydroborate, acyl β-alanine sodium, disodium hydrogen phosphate, sodium perchlorate monohydrate, (+)-sodium pantothenate, sodium metasilicate (anhydrous), sodium tetrafluoroborate, sodium sulfanylate dihydrate, sodium cellulose sulfate, pantoprazole sodium hydrate, sodium chondroitin sulfate, disodium methylarsonate, sodium hexafluorosilicate, riboflavin sodium phosphate, dextran sulfate sodium sulfur, trisodium phosphate dodecahydrate, disodium 5'-guanylate, disodium 5'-inosinate, dextran sulfate sodium sulfur 18, sulfobromophthalein sodium, disodium 5'-cytidylate, sodium lauryl sulfonate, betamethasone sodium phosphate, sodium polystyrene sulfonate, prednisolone sodium phosphate, sodium pentachlorophenolate, sodium dichloroisocyanurate, dexamethasone sodium phosphate, estramustine sodium phosphate, prednisolone sodium succinate, sodium hydrogen fumarate, sodium hexacyanoferrate (II), sodium 1-dodecanesulfonate, sodium dihydrogen phosphate dihydrate, carbazochrome sodium sulfonate, hydrocortisone sodium phosphate, sodium 2-hydroxypropanoate, sodium pyrophosphate decahydrate, disodium dihydrogenpyrophosphate, sodium triacetoxyborohydride, hydrocortisone sodium succinate, dibasic sodium phosphate dodecahydrate, colistin sodium methanesulfonate, sodium dodecylbenzene sulfonate, chloramphenicol sodium succinate, sodium 2-hydroxypropionate, sodium (Z)-9-octadecenoate, disodium 2-hydroxysuccinate, sodium cyclohexylsulfamate, flavin adenine dinucleotide sodium, disodium uridine-5'-diphosphate, sodium diphenylamine-4-sulfonate, methylprednisolone sodium succinate, an inosine 5'-monophosphate sodium salt, inosine 5'-phosphate disodium, sodium N-cyclohexylsulfamate, disodium cytidine 5'-phosphate, disodium guanosine-5'-phosphate, an (E)-2-butenedioate hydrogen 1-sodium salt, dexamethasone sodium metasulfobenzoate, sodium 2,4-dichlorophenoxyacetate, sodium (2,4-dichlorophenoxy)acetate, disodium 1,5-naphthalenedisulfonate, pentachlorophenol, sodium salt (for the industry), sodium 4-(2,4-dichloro-m-toluoyl)-1,3-dimethyl-pyrazole-5-olate, sodium 2-methyl-4-chlorophenoxyacetate, sodium (4-chloro-2-methylphenoxy)acetate, sodium [(4-chloro-2-methylphenyl)oxy]acetate, hardened coconut oil fatty acid glycerin sodium sulfate, sodium 5-ethyl-5,8-dihydro-8-oxofuro[3,2-b] [1,8]naphthyridine-7-carboxylate, trisodium 2-hydroxy-1,2,3-propane tricarboxylate, disodium 1-phenylazo-2-naphthol-4',6-disulfonate, sodium 1-(p-sodiosulfophenylazo)-2-hydroxynaphthalene-6-sulfonate, disodium 6-hydroxy-5-[(4-sulfophenyl)azo]-2-naphthalene sulfonate, trisodium 2-hydroxy(1,1'-azobisnaphthalene)-3,4',6-trisulfonate, (+)-N-[(R)-2,4-dihydroxy-3,3-dimethylbutyryl]-β-alanine sodium, disodium 4-[(2,4-dimethylphenyl)azo]-3-hydroxy-2,7-naphthalene disulfonate, sodium (+)-3-[[(R)-2,4-dihydroxy-3,3-dimethylbutyryl] amino]propionate, and sodium [methyl(2,3-dihydro-1,5-dimethyl-3-oxo-2-phenyl-1H-pyrazol-4-yl)amino]methanesulfo nate; potassium compounds such as potassium alum, potassium oxide, potassium bromide, potassium iodide, potassium nitrate, potassium sulfate, potassium sulfide, potassium acetate, potassium bromate, potassium chloride, potassium nitrite, potassium sulfite, potassium arsenate, potassium iodate, potassium hydride, potassium t-butoxide, potassium carbonate, potassium chlorate, potassium cyanate, potassium cyanide, potassium fluoride, potassium oxalate, potassium silicate, potassium arsenite, potassium stannate, potassium chromate, potassium hydroxide, potassium phosphate, potassium sorbate, potassium bisulfate, potassium selenate, potassium persulfate, potassium rhodanide, losartan potassium, potassium selenite, potassium tellurite, warfarin potassium, potassium periodate, potassium gluconate, potassium canrenoate, potassium perchlorate, potassium thiosulfate, potassium dichromate, potassium fluoroborate, potassium dichromate, potassium molybdate, potassium pyrosulfite, potassium aspartate, permirolast potassium, potassium hypochlorite, potassium thiocyanate, potassium clavulanate, aluminum potassium sulfate, potassium sulfocyanate, potassium borohydroxide, potassium hypophosphite, potassium metaphosphate, potassium permanganate, potassium pyrophosphate, potassium ferricyanide, potassium ferrocyanide, potassium silicofluoride, potassium hydrogen sulfate, potassium metavanadate, potassium xanthate, potassium hydrogen oxalate, clorazepate dipotassium, potassium selenocyanate, dipotassium oxalate, potassium fluorosilicate, potassium stannate trihydrate, potassium hydrogen carbonate, potassium peroxodisulfate, aluminum potassium sulfate, tetrapotassium pyrophosphate, benzylpenicillin potassium, potassium (methylsulfinyl)methanide, potassium dihydrogen phosphate, dipotassium hydrogenphosphate, dipotassium glycyrrhizate, potassium tetrafluoroborate, potassium guaiacolsulfonate, potassium hexafluorosilicate, aluminum potassium sulfate dodecahydrate, potassium o-ethyldithiocarbonate, potassium hexacyanoferrate (III), phenoxymethyl-penicillin potassium, and potassium (2E, 4E)-2,4-hexadienoate; rubidium compounds such as rubidium oxide, rubidium bromide, rubidium iodide, rubidium sulfate, rubidium chloride, rubidium fluoride, rubidium acetate, rubidium hydrogen sulfate, and rubidium carbonate; cesium compounds such as cesium cyanide, cesium oxide, cesium bromide, cesium iodide, cesium nitrate, cesium sulfate, cesium chloride, cesium carbonate, cesium fluoride, dicesium carbonate, dicesium oxalate, dicesium sulfate, dicesium L-tartrate, cesium hydroxide monohydrate, cesium trifluoroacetate, 2,3-dihydroxybutanedioic acid 1-cesium, and2,3-dihydroxybutanedioic acid hydrogen 1-cesium; beryllium compounds such as beryllium oxide, beryllium bromide, beryllium carbide, beryllium iodide, beryllium nitrate, beryllium nitride, beryllium sulfide, beryllium chloride, beryllium fluoride, beryllium silicate, beryllium distearate, a beryllium sulfate tetrahydrate salt, and an acetylacetone beryllium salt; magnesium compounds such as magnesium oxide, magnesium orotate, aryl magnesium, magnesium acetate, magnesium boride, magnesium bromide, magnesium carbide, magnesium iodide, magnesium nitrate, magnesium nitride, magnesium sulfate, magnesium sulfide, magnesium (powder), magnesium chloride, magnesium carbonate, magnesium chlorate, magnesium citrate, magnesium fluoride, magnesium silicate, magnesium silicide, magnesium hydroxide, magnesium phosphide, magnesium orotate, magnesium (II) nitrate, magnesium stearate, magnesium perchlorate, magnesium myristate, magnesium oxalate, magnesium hydrogen carbonate, magnesium silicofluoride, magnesium fluosilicate, phenyl magnesium bromide, magnesium nitrate hexahydrate, phthalocyanine magnesium, magnesium acetate tetrahydrate, magnesium benzoate trihydrate, calcium magnesium carbonate, magnesium chromate pentahydrate, magnesium hexafluorosilicate, magnesium aluminometasilicate, bismuth magnesium aluminosilicate, and aluminum magnesium hydroxide carbonate hydrate; calcium compounds such as calcium carbide, calcium oxide, calcium bromide, calcium carbide, calcium cyanamide, calcium iodide, calcium lactate, calcium nitrate, calcium nitride, calcium sulfate, calcium sulfide, calcium acetate, calcium chloride, calcium nitrite, calcium sulfite, calcium arsenate, calcium hydride, calcium arsenate, calcium iodate, calcium carbonate, calcium chlorate, calcium fluoride, calcium oxalate, calcium peroxide, calcium silicate, calcium silicide, calcium cyanide, calcium arsenite, heparin calcium, calcium chlorite, calcium chromate, calcium hydroxide, calcium phosphate, calcium phosphide, calcium folinate, calcium gluconate, calcium stearate, calcium aspartate, calcium hypochlorite, fosfomycin calcium, calcium metasilicate, calcium thiocyanate, calcium hypochlorite, calcium pantothenate, calcium permanganate, fenoprofen calcium, calcium chloride dihydrate, calcium magnesium carbonate, polycarbophil calcium, mupirocin calcium hydrate, calcium hydrogen phosphate, calcium glycerophosphate, calcium diiodostearate, precipitated calcium carbonate, calcium disodium edetate, atorvastatin calcium hydrate, (+)-calcium pantothenate, calcium para-aminosalicylate, tocopherol calcium succinate, calcium polystyrene-sulfonate, (+)-calcium pantothenate (2:1), calcium aluminoparaaminosalicylate, calcium 3-hydroxy-4-[(4-methyl-2-sulfophenyl)azo]-2-naphthalene-carboxylate; barium compounds such as barium oxide, barium azide, barium acetate, barium bromide, barium iodide, barium nitrate, barium nitride, barium sulfate, barium sulfide, barium formate, barium lactate, barium chloride, barium sulfite, barium carbonate, barium chlorate, barium fluoride, barium peroxide, barium cyanide, barium chromate, barium hydroxide, barium titanate, barium selenate, barium phosphate, barium (II) nitrate, barium selenite, barium manganate, barium oxalate, barium stearate, barium perchlorate, barium thiosulfate, barium hypochlorite, barium permanganate, barium chloranilate, barium chloride dihydrate, barium diacetate, barium bromide dihydrate, barium dipropionate, barium chlorate monohydrate, barium hydroxide octahydrate, barium thiocyanate dihydrate, barium thiosulfate monohydrate, and acetylacetone barium dihydrate; and the like.

Examples of the unsaturated monomer include hydrophilic unsaturated monomers containing an acid group such as an acrylic acid-based polymer, maleic acid, maleic anhydride, fumaric acid, crotonic acid, itaconic acid, vinyl sulfonic acid, styrene sulfonic acid, 2-(meth)acrylamido-2-methylpropane sulfonic acid, 2-(meth)acryloylethane sulfonic acid, and 2-(meth)acryloylpropane sulfonic acid, and a salt thereof; nonionic hydrophilic unsaturated monomers such as N-vinylacetamide, N-methyl-N-vinylacetamide, acrylamide, methacrylamide, N-ethyl (meth)acrylamide, N-n-propyl (meth)acrylamide, N-isopropyl (meth)acrylamide, N,N-dimethyl (meth)acrylamide, 2-hydroxyethyl (meth)acrylate, 2-hydroxypropyl (meth)acrylate, methoxypolyethylene glycol (meth)acrylate, polyethylene glycol mono(meth)acrylate, vinyl pyridine, N-vinylpyrrolidone, N-acryloyl piperidine, and N-acryloyl pyrrolidine; cationic hydrophilic unsaturated monomers such as N,N-dimethylaminoethyl (meth)acrylate, N,N-diethylaminoethyl (meth)acrylate, N,N-dimethylaminopropyl (meth)acrylate, and N,N-dimethylaminopropyl (meth)acrylamide, and quaternary salts thereof, styrene, vinyl chloride, butadiene, isobutene, ethylene, propylene, stearyl (meth)acrylate, and lauryl (meth)acrylate.

Examples of the polyhydric alcohol include ethylene glycol, propylene glycol, 1,3-butylene glycol, ethylene glycol monobutyl ether, diethylene glycol, triethylene glycol, 1,4-butylene glycol (dihydric alcohol), glycerin, trioxyisobutane (trihydric alcohol), erythrite, pentaerythrite (tetrahydric alcohol), xylit, adonit (pentahydric alcohol), allodulcite, sorbitol, sorbitol liquid, mannitol (hexahydric alcohol), polyglycerin, and dipropylene glycol.

Examples of the polymer additive include polyvinyl pyrrolidone, a carboxy vinyl polymer which is a cross-linked polyacrylic acid, a vinyl pyrrolidone-ethyl acrylate copolymer, an N-vinyl acetamide-based copolymer such as an N-vinyl acetamide-sodium acrylate copolymer, an N-vinyl acetamide homopolymer, polyvinyl sulfonic acid, an N-vinyl acetamide cross-linked product, polyitaconic acid, hydroxypropyl cellulose, and hydroxypropyl methylcellulose.

Examples of the antiinflammatory analgesic agent include salicylic acid, glycol salicylate, methyl salicylate, 1-menthol, camphor, sulindac, trimethine sodium, naproxen, fenbufen, piroxicam, triamcinolone, hydrocortisone acetate, indomethacin, ketoprofen, acetaminophen, mefenamic acid, flufenamic acid, ibufenac, loxoprofen, tiaprofen, pranoprofen, fenbufen, diclofenac, diclofenac sodium, alclofenac, lornoxicam, pranoprofen, oxyphenbutazone, ibuprofen, felbinac, ketorolac, bermoprofen, nabumetone, naproxen, flurbiprofen, fluocinonide, clobetasol propionate, COX-2 inhibitors (celecoxib, rofecoxib, varecoxib, parecoxib, and valdecoxib), etodolac, nimesulide, and meloxicam.

Examples of the antifungal agent include clotrimazole, econazole nitrate, omoconazole nitrate, tioconazole nitrate, ketoconazole nitrate, miconazole nitrate, isoconazole nitrate, tolnaftate, sulconazole nitrate, pyrrolnitrin, pimafucin, undecylenic acid, salicylic acid, siccanin, nystatin, nornaftate, exalamide, phenyliodoundecynoate, thianthol, cyclopiroxolamine, haloprogin, trichomycin, variotin, pentamycin, and amphotericin B.

Examples of the antihistamine agent include antibiotics such as tetracycline hydrochloride, diphenhydramine hydrochloride, chlorpheniramine, diphenyl imidazole, and chloramphenicol, diphenhydramine, chlorpheniramine maleate, diphenhydramine hydrochloride, chlorpheniramine, and diphenylimidazole.

Examples of the hypnotic sedative include phenobarbital, amobarbital, cyclobarbital, lorazepam, and haloperidol.

Examples of the tranquilizer include fluphenazine, thioridazine, diazepam, flunitrazepam, and chlorpromazine.

Examples of the antihypertensive agent include clonidine, clonidine hydrochloride, pindolol, propranolol, propranolol hydrochloride, bufuralol, indenolol, bucumolol, and nifedipine.

Examples of the hypotensive diuretic include hydrothiazide and cyclopenthiazide.

Examples of the antibiotic include penicillin, tetracycline, oxytetracycline, fradiomycin sulfate, erythromycin, and chloramphenicol.

Examples of the anesthetic include lidocaine, benzocaine, ethyl aminobenzoate, and dibucaine.

Examples of the antibacterial substance include benzalkonium chloride, nitrofurazone, nystatin, acetosulfamine, and clotrimazole.

Examples of the antiepileptic agent include nitrazepam, meprobamate, and clonazepam.

Examples of the coronary vasodilator include nitroglycerin, nitroglycol, isosorbide nitrate, erythritol tetranitrate, pentaerythritol tetranitrate, and propatyl nitrate.

Examples of the herbal medicine include cork tree bark, cherry tree, polygala root, zedoary, chamomile, trichosanthes seed, licorice, platycodon root, apricot kernel, bezoar, schisandra fruit, honey locust, bupleurum root, asiasarum root, plantago seed shazenshi, cimicifugae rhizoma, senega, atractylodes lancea rhizome, mulberry root bark, clove, citrus unshiu peel, ipecac, nandina domestica fruit, fritillaria bulb, ophiopogon tuber, pinellia tuber, atractylodes rhizome, henbane, ledebouriella seseloides, and ephedra.

Examples of the auxiliary agent include keratin layer softeners such as ethyl alcohol, isopropyl alcohol, butanol, 1,3-butanediol, propylene glycol, polyethylene glycol # 400, glycerin, crotamiton, benzyl alcohol, phenyl ethyl alcohol, propylene carbonate, hexyl dodecanol, propanol, salicylic acid, allantoin, dimethyl sulfoxide, dimethyl acetamide, dimethyl formamide, diisopropyl adipate, diethyl sebacate, ethyl laurate, lanoline, azone, 1-geranylazacycloheptan-2-one (GACH), dialkylolamide of a fatty acid, salicylic acid, salicylic acid derivatives, urea, and sulfur, moisturizing agents such as pyrrolidone carboxylic acid, surfactants such as propylene glycol monooleate, polyoxyethylene sorbitan monostearate, sorbitan monostearate, and glycerin monostearate, esters such as isopropyl myristate and diethyl sebacate, higher alcohols such as oleyl alcohol, stearyl alcohol, and lauryl alcohol, fatty acids such as stearic acid, hexanoic acid, nonanoic acid, decanoic acid, dodecanoic acid, tetradecanoic acid, hexadecanoic acid, octadecanoic acid, oleic acid, and linoleic acid, terpene-based compounds and surfactants such as menthol, menthone, limonene, pinene, piperitone, terpinene, terpinolene, terpinolol, and carveol, assistants such as allantoin, dimethyl sulfoxide, dimethylacetamide, dimethyl formamide, diisopropyl adipate, diethyl sebacate, ethyl laurate, lanolin, and azone, and, as necessary, cooling agents such as menthol and camphor, oily components such as almond oil, olive oil, camellia oil, persic oil, peppermint oil, sesame oil, soybean oil, mink oil, cottonseed oil, corn oil, safflower oil, coconut oil, eucalyptus oil, castor oil, liquid paraffin, vaseline, squalene, squalane, and lanolin, gelling agents such as a carboxyvinyl polymer, and neutralizers such as diisopropanolamine.

Examples of the wetting agent include glycerine, propylene glycol, sorbitol, 1,3-butylene glycol, dl-pyrrolidone carboxylic acid, and sodium lactate.

Examples of the astringent agent include citric acid, tartaric acid, lactic acid, aluminum chloride, aluminum sulfate, allantoin chlorohydroxy aluminum, allantoin dihydroxy aluminum, aluminum phenolsulfonate, zinc para-phenolsulfonate, zinc sulfate, and aluminum chlorohydroxide.

Examples of the thickening agent include natural polymers such as gum arabic, tragacanth, locust bean gum, guar gum, xanthan gum, karaya gum, agar, starch, carrageenan, alginic acid, alginic acid salts (for example, sodium alginate), propylene glycol alginate, dextran, dextrin, amylose, gelatin, collagen, pullulan, pectin, amylopectin, starch, sodium amylopectin semi-glycolate, chitin, albumin, and casein, semi-synthetic polymers such as polyglutamic acid, polyaspartic acid, methyl cellulose, ethyl cellulose, propyl cellulose, ethyl methyl cellulose, hydroxyethyl cellulose, hydroxypropyl cellulose, hydroxypropyl methyl cellulose, carboxymethyl starch, alkali metal carboxymethyl cellulose, alkali metal cellulose sulfate, a cellulose graft polymer, cross-linked gelatin, cellulose acetate phthalate, a starch-acrylic acid graft polymer, phthalic anhydride-modified gelatin, and succinic acid-modified gelatin, polyvinyl alcohol, polyvinylpyrrolidone, polyvinyl methyl ether, a carboxyvinyl polymer, a vinylpyrrolidone-ethyl acrylate copolymer, a vinylpyrrolidone-styrene copolymer, a vinylpyrrolidone-vinyl acetate copolymer, a vinyl acetate-(meth)acrylic acid copolymer, a polyvinyl acetate-crotonicacid copolymer, N-vinylacetamide-based copolymers such as an N-vinylacetamide-sodium acrylate copolymer, polyvinyl sulfonate, an N-vinylacetamide cross-linked product, polyitaconic acid, polyhydroxyethyl acrylate, polyacrylamide, a styrene-maleic anhydride copolymer, and an acrylamide-acrylic acid copolymer.

Examples of the tackifying material include silicone rubber, polyisoprene rubber, styrene-block copolymer rubber, acrylic rubber, and natural rubber.

Examples of the antipruritic agent include camphor, thymol, menthol, polyoxyethylene lauryl ether, an antihistamine, and ethyl aminobenzoate.

Examples of the keratin layer softening and peeling agent include sulfur, thioxolone, selenium sulfide, salicylic acid, and resorcinol.

Examples of the oily material include almond oil, olive oil, hard oil, camellia oil, castor oil, Japan wax oil, coconut oil, beeswax, spermacetieti, lanolin, carnauba wax, candelilla wax, liquid paraffin, vaseline, microcrystalline wax, ceresine, squalene, lauric acid, myristic acid, palmitic acid, stearic acid, isostearic acid, oleic acid, lauryl alcohol, cetanol, stearyl alcohol, oleyl alcohol, octyl dodecanol, cholesterol, hexyl decanol, white sterol, cetyl lactate, isopropyl myristate, hexyl laurate, myristyl myristate, isopropyl palmitate, octyl myristate dodecanol, butyl stearate, cacao oil, Japan wax, jojoba oil, grape seed oil, avocado oil, mink oil, egg yolk oil, beeswax, spermacetieti, lanolin, carnauba wax, candelilla wax, liquid paraffin, ceresine wax, paraffin wax, behenic acid, isopropyl adipate, octyldodecyl myristate, octyldodecyl oleate, and cholesterol oleate.

Examples of the ultraviolet screening agent include benzophenone-based ultraviolet screening agents such as ASL-24, Cyasorb UV-9, and Uvinul M-40, benzoic acid-based ultraviolet screening agents such as Salol, azole-based ultraviolet screening agents such as Tinuvin P, nitrile-based ultraviolet screening agents such as Uvinul N-35, urea-based ultraviolet screening agents such as Ancour UA, para-amino acid-based ultraviolet screening agents such as Neo Heliopan Give tan F, 2-hydroxy-4-methoxybenzophenone, octyldimethyl para-amino benzoate, and ethylhexyl para-methoxycinnamate, a salicylic acid-based ultraviolet screening agent, a benzofuran-based ultraviolet screening agent, a coumarin-based ultraviolet screening agent, and an azole-based ultraviolet screening agent.

Examples of the antiseptic preservative include acids such as benzoic acid, salicylic acid, dehydroacetic acid, sorbic acid, and boric acid, and salts thereof, phenols such as phenol, chlorocresol, chloroxylenol, isopropyl methyl phenol, resorcinol, orthophenyl phenol, para-oxybenzoic acid ester, phenoxyethanol, thymol, hinokitiol, and thioxolone, halogenated bisphenols such as hexachlorophene and 2,4,4'-trichloro-2'-hydroxydiphenyl ether, amide compounds such as trichlorocarbanilide, halocarban, and undecylenic acid monoethanolamide, quaternary ammonium compounds such as benzalkonium chloride, alkyl isoquinolinium bromide, benzethonium chloride, and cetylpyridinium chloride, amphoteric surfactants such as lauryl di(aminoethyl)glycine, a 2-pyridine thiol-1-oxide zinc salt, gluconic acid, chlorhexidine, thiram, N-trichloromethyl thio-4-cyclohexene-1,2-dicarboximide, and chlorobutanol.

Examples of the antioxidant include methyl L-ascorbate, ethyl L-ascorbate, propyl L-ascorbate, butyl L-ascorbate, pentyl L-ascorbate, L-ascorbyl 2-palmitate, L-ascorbyl-2-isopalmitate, L-ascorbyl-2-stearate, L-ascorbyl-2-isostearate, L-ascorbyl-6-palmitate, L-ascorbyl-6-isopalmitate, L-ascorbyl-6-stearate, L-ascorbyl-6-isostearate, L-ascorbyl-2,6-dipalmitate, L-ascorbyl-2,6-diisopalmitate, L-ascorbyl-2,6-distearate, L-ascorbyl-2,6-diisostearate, L-ascorbyl-2-glucoside-6-palmitate, L-ascorbyl-2-glucoside-6-isopalmitate, L-ascorbyl-2-glucoside-6-stearate, L-ascorbyl-2-glucoside-6-isostearate, L-ascorbyl-2-glucoside-6-tocopheryl, L-ascorbyl-2-sulfate-6-palmitate, L-ascorbyl-2-sulfate-6-isopalmitate, L-ascorbyl-2-sulfate-6-stearate, L-ascorbyl-2-sulfate-6-isostearate, L-ascorbyl-2-sulfate-6-tocopheryl, L-ascorbyl-2-maleate-6-palmitate, L-ascorbyl-2-maleate-6-isopalmitate, L-ascorbyl-2-maleate-6-stearate, L-ascorbyl-2-maleate-6-isostearate, L-ascorbyl-2-maleate-6-tocopheryl, L-ascorbyl-2-phosphate-6-palmitate, L-ascorbyl-2-phosphate-6-isopalmitate, L-ascorbyl-2-phosphate-6-stearate, L-ascorbyl-2-phosphate-6-isostearate, L-ascorbyl-2-phosphate-6-tocopheryl, palmitoyl 2-0-L-ascorbic aicid, isopalmitoyl 2-0-L-ascorbyl phosphate, stearyl 2-0-L-ascorbyl phosphate, isostearyl 2-0-L-ascorbyl phosphate, tocopheryl 2-0-L-ascorbyl phosphate, retinoyl 2-0-L-ascorbyl phosphate, tocopheryl phosphate, tocotrienol phosphate, tocopheryl glucoside, tocopheryl sulfate, tocopheryl dimethyl glycine, retinoyl phosphate, retinoyl glucoside, retinoyl sulfate, and retinoyl dimethyl glycine.

Examples of the liquid matrix include glycerin, polyhydric alcohol, ethanol, propanol, isopropanol, a gelling agent, glycerin, methyl laurate, lauryl alcohol, glycerol monolaurate, oleic acid, oleyl alcohol, glycerol monooleate, glycerol dioleate, and glycerol trioleate, nicotinic acid, 2-pyrazine carboxylic acid, sinapic acid (3,5-dimethoxy-4-hydroxycinnamic acid), 2,5-dihydroxybenzoic acid, 5-methoxysalicylic acid, α-cyano-4-hydroxycinnamic acid, 3-hydroxy picolinic acid, diaminonaphthalene, 2-(4-hydroxyphenylazo)benzoic acid, dithranol, succinic acid, 5-(trifluoromethyl)uracil, propylene glycol, dipropylene glycol, polypropylene glycol, glycerin, diglycerin, polyglycerin, ethylene glycol, diethylene glycol, polyethylene glycol, 1,3-butanediol, sorbitol, maltitol, erythritol, pentaerythritol, glucose, saccharose, maltose, xylose, and trehalose.

Examples of the fat-soluble material include ascorbyl isopalmitate, ascorbyl-2-phosphate-6-palmitate, ascorbyl-2-phosphate-6-isopalmitate, ascorbyl tetrahexyldecanoate, ascorbyl-6-palmitate, ascorbyl-2,6-dipalmitate, ascorbyl-6-stearate, retinol, retinoic acid, retinol palmitate, tocopheryl retinoate, retinyl palmitate, retinyl isopalmitate, retinyl stearate, retinol stearate, astaxanthin, lutein, lycopene, β-carotene, α-carotene, α-tocopherol, β-tocopherol, γ-tocopherol, dimethyl glycine tocopherol, ubiquinone, coenzyme A, tocotrienol, mineral oil, liquid paraffin, squalane, isopropyl palmitate, isopropyl myristate, isooctyl myristate, isotridecyl myristate, octadecyl myristate, octyldodecyl myristate, isostearyl cholesteryl ester, triglyceride 2-ethylhexanoate, cetyl 2-ethyl hexanoate, sunflower oil, olive oil, jojoba oil, camellia oil, grape seed oil, avocado oil, macadamia nut oil, almond oil, rice germ oil, clove oil, orange oil, orange peel oil, pycnogenol, coenzyme A, iris extract oil, ashitaba extract oil, thujopsis dolobrata extract oil, asparagus extract oil, avocado extract oil, sweet hydrangea leaf extract oil, almond extract oil, althea extract oil, arnica extract oil, aloe extract oil, apricot extract oil, apricot kernel extract oil, ginkgo extract oil, inchikow extract oil, fennel extract oil, turmeric extract oil, oolong tea extract oil, uva ursi extract oil, rose fruit extract oil, echinacea leaf extract oil, isodon japonicus extract oil, scutellaria root extract oil, phellodendron bark extract oil, coptis japonica extract oil, barley extract oil, panax ginseng extract oil, hypericum extract oil, lamium album extract oil, ononis spinosa extract oil, Netherland mustard extract oil, orange extract oil, a dried seawater product, seaweed extract oil, persimmon leaf extract oil, pyracantha fortuneana fruit extract oil, hydrolyzed elastin, a hydrolyzed wheat powder, hydrolyzed silk, pueraria root extract oil, chamomilla extract oil, carrot extract oil, capillary artemisia extract oil, wild oat extract oil, hibiscus sabdariffa extract oil, licorice extract oil, oil-soluble licorice extract oil, kiwi extract oil, kiou apple extract oil, Jew's ear mushroom extract oil, cinchona bark extract oil, cucumber extract oil, paulownia tomentosa leaf extract oil, guanosine, guava extract oil, sophora root extract oil, gardenia extract oil, kuma bamboo grass extract oil, sophora flavescens extract oil, walnut extract oil, chestnut extract oil, grapefruit extract oil, clematis extract oil, black rice extract oil, fat-soluble brown sugar extract, fat-soluble black vinegar extract, chlorella extract oil, mulberry extract oil, gentian extract oil, geranium thumbergii extract oil, black tea extract oil, yeast extract oil, magnolia bark extract oil, coffee extract oil, burdock extract oil, rice extract oil, fermented rice extract oil, fermented rice bran extract oil, rice germ oil, comfrey extract oil, collagen, cowb extract oil, saisin extract oil, bupleurum root extract oil, umbilical cord extract liquid, saffron extract oil, salvia extract oil, saponaria officinalis extract oil, sasa extract oil, hawthorn extract oil, sansha extract oil, Japanese pepper extract oil, shiitake mushroom extract oil, rehmannia root extract oil, lithospermum root extract oil, perilla extract oil, linden extract oil, meadowsweet extract oil, jatoba extract oil, peony extract oil, ginger extract oil, calamus root extract oil, white birch extract oil, white Jew's ear mushroom extract oil, horsetail extract oil, stevia extract oil, a stevia-fermented product, Chinese tamarisk extract oil, English ivy extract oil, whitethorn extract oil, Sambucus nigra extract oil, yarrow extract oil, peppermint extract oil, sage extract oil, mallow extract oil, cnidium rhizome extract oil, Japanese green gentian extract oil, mulberry bark extract oil, rhubarb extract oil, soybean extract oil, jujube extract oil, thyme extract oil, dandelion extract oil, lichens extract oil, tea extract oil, clove extract oil, imperata extract oil, citrus unshiu peel extract oil, tea tree oil, Chinese blackberry extract oil, capsicum extract oil, Japanese angelica root extract oil, calendula officinalis extract oil, peach kernel extract oil, spruce extract oil, houttuynia extract oil, tomato extract oil, natto extract oil, carrot extract oil, garlic extract oil, eglantine extract oil, hibiscus extract oil, ophiopogon extract oil, lotus extract oil, parsley extract oil, birch extract oil, honey, hamamelis extract oil, parietaria extract oil, isodon japonicus extract oil, bisabolol, cypress extract oil, Lactobacillus bifidus extract oil, loquat extract oil, coltsfoot extract oil, petasites japonicus extract oil, hoelen extract oil, butcher bloom extract oil, grape extract oil, grape seed extract oil, propolis, loofah extract oil, safflower extract oil, peppermint extract oil, linden extract oil, tree peony extract oil, hop extract oil, rosa rugosa extract oil, pine extract oil, horse chestnut extract oil, skunk cabbage extract oil, soapberry extract oil, melissa extract oil, mozuku extract oil, peach extract oil, cornflower extract oil, eucalyptus oil extract, saxifrage extract oil, yuzu extract oil, lily extract oil, coix seed extract oil, wormwood extract oil, lavender extract oil, green tea extract oil, egg shell membrane extract oil, apple extract oil, rooibos tea extract oil, litchi extract oil, lettuce extract oil, lemon extract oil, weeping forsythia extract oil, astragalus sinicus extract oil, rose extract oil, rosemary extract oil, roman chamomile extract oil, royal jelly extract oil, burnet extract oil, octamethyl trisiloxane, decamethyl tetrasiloxane, methyl polysiloxane, dimethyl silicone oils such as highly polymerized methyl polysiloxane; cyclic silicone oils such as octamethyl cyclotetrasiloxane, decamethyl cyclopentasiloxane, dodecamethyl cyclohexasiloxane, methyl cyclopolysiloxane, and methyl polycyclosiloxane; and methyl phenyl silicone oils such as polyether-modified silicone oil and methyl phenyl polysiloxane.

Examples of the polymer carboxylate include acrylic resins such as polyacrylic acid, an acrylic acid-acrylonitrile copolymer, a potassium acrylate-acrylonitrile copolymer, a vinyl acetate-acrylic acid ester copolymer, and an acrylic acid-acrylic acid alkyl ester copolymer, styrene acrylic resins such as a styrene-acrylic acid copolymer, a styrene-methacrylic acid copolymer, a styrene-methacrylic acid-acrylic acid alkyl ester copolymer, a styrene-α-methyl styrene-acrylic acid copolymer, and a styrene-α-methyl styrene-acrylic acid-acrylic acid alkyl ester copolymer; a styrene-maleic acid copolymer and a styrene-maleic anhydride copolymer; vinyl acetate resins such as a vinyl naphthalene-acrylic acid copolymer, a vinyl acetate-ethylene copolymer, a vinyl acetate-fatty acid vinyl ethylene copolymer, a vinyl acetate-maleic acid ester copolymer, a vinyl acetate-crotonic acid copolymer, and a vinyl acetate-acrylic acid copolymer; and a salt of any polyester or cellulose polymer.

Examples of the additive include hydrocarbons, waxes, fatty acids, higher alcohols, ester oils, silicone oils, fluorine-based oils, that is, hydrocarbons such as ozokerite, squalane, squalene, ceresine, paraffin, paraffin wax, liquid paraffin, pristane, polyisobutylene, microcrystalline wax, and vaseline, waxes such as beeswax, carnauba wax, candelilla wax, and spermacetieti, animal oils such as beef tallow, neatsfoot oil, beef bone fat, hard beef tallow oil, hard oil, turtle oil, lard, horse fat, mink oil, cod-liver oil, and egg yolk oil, lanolin derivatives such as lanolin, liquid lanolin, reduced lanolin, lanolin alcohol, a hard lanolin, lanolin acetate, fatty acid lanolin isopropyl, phospholipid, phosphatidylcholine, POE lanolin alcohol ether, POE lanolin alcohol acetate, fatty acid lanolin polyethylene glycol, and POE hydrogenated lanolin alcohol ether, fatty acids such as lauric acid, myristic acid, palmitic acid, stearic acid, behenic acid, undecylenic acid, oleic acid, linoleic acid, arachidonic acid, eicosapentaenoic acid (EPA), docosahexaenoic acid (DHA), isostearic acid, and 12-hydroxy stearic acid, higher alcohols such as lauryl alcohol, myristyl alcohol, palmityl alcohol, stearyl alcohol, behenyl alcohol, hexadecyl alcohol, oleyl alcohol, isostearyl alcohol, hexyl dodecanol, octyl dodecanol, cetostearyl alcohol-2-decyltetradecinol, cholesterol, phytosterol, sitosterol, lanosterol, POE cholesterol ether, and monostearyl glycerin ether (batyl alcohol), ester oils such as adipic acid diisobutyl ester, adipic acid 2-hexyl decyl ester, adipic acid di-2-heptylundecyl ester, monoisostearic acid N-alkyl glycol ester, isostearic acid isocetyl ester, triisostearic acid trimethylol propane ester, di-2-ethylhexanoic acid ethylene glycol 2-ethylhexanoic acid cetyl ester, tri-2-ethylhexanoic acid trimethylol propane ester, tetra-2-ethylhexanoic acid pentaerythritol ester, octanoic acid cetyl ester, octyldodecyl gum ester, oleic acid oleyl ester, oleic acid octyldodecyl ester, oleic acid decyl ester, dicapric acid neopentyl glycol ester, cirtic acid triethyl ester, succinic acid 2-ethylhexyl ester, acetic acid amyl ester, acetic acid ethyl ester, acetic acid butyl ester, stearic acid isocetyl ester, stearic acid butyl ester, sebacic acid diisopropyl ester, sebacic acid di-2-ethylhexyl ester, lactic acid cetyl ester, lactic acid myristyl ester, palmitic acid isopropyl ester, palmitic acid 2-ethylhexyl ester, palmitic acid 2-hexyldecyl ester, palmitic acid 2-heptylundecyl ester, 12-hydroxy stearic acid cholesteryl ester, dipentaerythritol fatty acid ester, myristic acid isopropyl ester, myristic acid octyldodecyl ester, myristic acid 2-hexyldecyl ester, myristic acid myristyl ester, dimethyl octanoic acid hexyldecyl ester, lauric acid ethyl ester, lauric acid hexyl ester, N-lauroyl-L-glutamic acid 2-octyldodecyl ester, and malic acid diisostearyl ester, glyceride oils such as acetoglyceride, triisooctanoic acid glyceride, triisostearic acid glyceride, triisopalmitic acid glyceride, tri-2-ethyl hexanoic acid glyceride, monostearic acid glyceride, di-2-heptyl undecanoic acid glyceride, and trimyristic acid glyceride, higher alkoxy-modified silicones such as dimethyl polysiloxane, methylphenyl polysiloxane, methylhydrogen polysiloxane, octamethyl cyclotetrasiloxane, decamethyl cyclopentasiloxane, dodecamethyl cyclohexasiloxane, tetramethyl tetrahydrogen cyclotetrasiloxane, and stearoxy silicone, silicone oils such as higher fatty acid-modified silicone, a silicone resin, silicon rubber, and silicone resin, and fluorine-based oils such as perfluoropolyether, perfluorodecalin, and perfluorooctane.

Examples of the metal soap include aluminum 12-hydroxystearate, zinc stearate, aluminum stearate, calcium stearate, magnesium stearate, zinc myristate, magnesium myristate, zinc cetyl phosphate, calcium cetyl phosphate, sodium zinc cetyl phosphate, zinc laurate, and zinc undecylenate.

Examples of the anti-aging composition of the present invention include a cosmetic, a skin external agent, a medicine, and the like.

In a case where the anti-aging composition of the present invention is a cosmetic, it is possible to further add existing cosmetic raw material in a general concentration. For example, all cosmetic raw materials described in the Japanese Standards of Cosmetic Ingredients, 2nd edition commentary edited by Pharmaceutical and Medical Device Regulatory Science Society of Japan, 1984 (Yakuji Nippo Ltd.), Supplement to The Japanese Cosmetic Ingredients Codex, Ministry of Health and Welfare Pharmaceutical Affairs Bureau examination Division supervision, 1993 (Yakuji Nippo Ltd.), Supplement to The Japanese Cosmetic Ingredients Codex, Supplement, Ministry of Health and Welfare Pharmaceutical Affairs Bureau examination Division supervision, 1993 (Yakuji Nippo Ltd.), The Comprehensive Licensing Standards of Cosmetics by Category, Ministry of Health and Welfare Pharmaceutical Affairs Bureau examination Division supervision, 1993 (Yakuji Nippo Ltd.), Japanese Cosmetic Ingredients Codex, Ministry of Health and Welfare Pharmaceutical Affairs Bureau examination Division supervision, 1997 (Yakuji Nippo Ltd.), and Cosmetic Ingredients Dictionary, 1991 (Nikko Chemicals) can be used.

As examples of the kinds of the anti-aging composition of the present invention, it is possible to apply a hair cosmetic selected from shampoo, oil shampoo, cream shampoo, conditioning shampoo, shampoo for dandruff, shampoo for hair color, rinse integrated shampoo, rinse, treatment, hair pack, hair foam, hair mousse, hair spray, hair mist, hair wax, hair gel, water grease, setting lotion, color lotion, hair tonic, hair liquid, pomade, chick, hair cream, hair blow, split hair coater, hair oil, agent for permanent wave, straight permanent treatment agent, oxidation hair dye, hair bleach, hair color pretreatment, hair color after treatment, permanent pretreatment, permanent after treatment, hair manicure, and hair growth tonic; basic cosmetic selected from facial cleanser, cleansing foam, soap powder, facial cleansing powder, cleansing cream, cleansing milk, cleansing lotion, cleansing gel, cleansing oil, cleansing mask, lotion, softening lotion, astringent lotion, lotion for cleansing, multilayered lotion, emulsion, emollient lotion, moisture lotion, milky lotion, nourishing lotion, nourishing milk, skin moisturizer, moisture emulsion, massage lotion, cleansing lotion, protection emulsion, sun protection, sun protector, UV care milk, sun screen, makeup lotion, keratin smoother, elbow lotion, hand lotion, body lotion, cream, emollient cream, nutritive cream, nourishing cream, vanishing cream, moisture cream, night cream, massage cream, cleansing cream, makeup cream, base cream, pre-makeup cream, sunscreen cream, suntan cream, hair removal cream, deodorant cream, shaving cream, keratin layer softening cream, gel, cleansing gel, moisture gel, soap, toilet soap, transparent soap, medicated soap, liquid soap, shaving soap, synthetic toilet soap, pack, mask, peel off pack, powder pack, washing pack, oil pack, cleansing mask, essence, moisturizing essence, whitening essence, UV protection essence, liposome essence, and liposome lotion; makeup cosmetic selected from face powder, dusting powder, foundations, makeup base, lipsticks, lip gloss, rouges, eyeliner, mascara, eye shadow, eyebrow pencil, eyebrow, nail enamel, enamel remover, nail treatment; fragrant cosmetic selected from perfume, perfume, parfum, eau de parfum, eau de toilette, eau de cologne, solid perfume, aromatic powder, perfume soap, body lotion, and bath oil; and body cosmetics selected from body shampoo, body cleanser, body powder, deodorant lotion, deodorant powder, deodorant spray, deodorant stick, deodorant cosmetics, bleaching agent, depilation-epilation agent, bath agent, bug repellent spray, and insect repeller; ointment, patch, lotion, liniment, and liquid coating agent.

The dosage form of the anti-aging composition of the present invention is not particularly limited as long as the composition is in contact with the skin while being used, and can be appropriately set according to the usage. As dosage form, it is possible to apply, for example, emulsion forms such as oil-in-water (O/W) form, water-in-oil (W/O) form, W/O/W form, and O/W/O form, emulsion polymer form, oily form, solid form, liquid form, paste form, stick form, volatile oil form, powder form, jelly form, gel form, paste form, cream form, sheet form, film form, mist form, spray form, multi-layered form, foam form, and flake form.

The anti-aging composition of the present invention preferably has a pH of 6 to 9, more preferably a pH of 7 to 8.5. When a pH is within this range, stability of a hydroxycitric acid derivative is good, and therefore preparation stability also becomes good. These pHs are values at approximately 25°C.

For a blending amount of the hydroxycitric acid derivative or a salt thereof contained in the anti-aging composition of the present invention, for example, approximately 0.01% by mass to 20% by mass is preferable, approximately 0.05% by mass to 10% by mass is more preferable, and approximately 0.1% by mass to 5% by mass is further preferable as the ratio (% by mass) of the hydroxycitric acid derivative or a salt thereof in the anti-aging composition.

It is considered that the anti-aging composition of the present invention exhibits an effect on activating cells and improving cell functions, the effect being achieved by the effect of the agent for improving mitochondrial function on improving mitochondrial function in cells, and therefore is effective in preventing or improving aging of cells and the skin, such as the formation of wrinkles, sagging, and the like.

In another embodiment, the present invention provides a method for preventing cell aging, comprising bringing an effective amount of a hydroxycitric acid derivative or a salt thereof into contact with cells to be cultured.

In still another embodiment, the present invention provides a hydroxycitric acid derivative or a salt thereof for preventing or improving skin aging.

In still further another embodiment, the present invention provides the use of a hydroxycitric acid derivative or a salt thereof for preventing or improving skin aging.

In still further another embodiment, the present invention provides the use of a hydroxycitric acid derivative or a salt thereof for producing an anti-aging composition.

In still further another embodiment, the present invention provides a method for preventing or improving skin aging, comprising bringing an effective amount of a hydroxycitric acid derivative or a salt thereof into contact with the skin of a patient in need of treatment.

The prevention and improvement of skin aging can be confirmed by a method described in Examples below with an amount of elastin produced by cells as an index. For example, a cell group brought into contact with the anti-aging composition and a cell group not brought into contact with the anti-aging composition are compared, and in a case where an amount of elastin produced by cells is higher in the cell group brought into contact with the anti-aging composition, it can be determined that the anti-aging composition has an effect on preventing and improving skin aging.

The prevention and improvement of skin aging can be confirmed by a method described in Examples below with a state of the skin as an index. For example, skin brought into contact with the anti-aging composition and skin not brought into contact with the anti-aging composition are compared, and in a case where any difference in aspects such as a higher amount of skin moisture, higher level of skin smoothness, or higher level of skin elasticityoccurs in the skin brought into contact with the anti-aging composition, it can be determined that the anti-aging composition has an effect on preventing and improving skin aging.

### Examples

Hereinafter, the present invention will further be described in detail based on Examples, but the present invention is not limited to the following Examples.

### [Example 1]

Preparation comprising a hydroxycitric acid derivative according to the present invention can be performed according to formulations shown in the following (Table 1) as Formulation Example.

### Formulation Example 1 Cream

[Table 1]

**(Table 1)**

| | | Formulation 1 | Formulation 2 | Formulation 3 |
|---|---|---|---|---|
| A | Hydroxycitric acid-2-palmitate | 1.0 | - | - |
| | Hydroxycitric acid-2-myristate | - | 1.0 | - |
| | Hydrogenated rapeseed oil alcohol | 4.2 | 4.2 | 4.2 |
| | Isononyl isononanoate | 6.0 | 6.0 | 6.0 |
| | Squalane | 9.6 | 9.6 | 9.6 |
| | Octyldodecyl myristate | 4.8 | 4.8 | 4.8 |
| | Polyglyceryl-10 stearate | 2.0 | 2.0 | 2.0 |
| | Glyceryl stearate | 1.0 | 1.0 | 1.0 |
| | Tocopherol | 0.2 | 0.2 | 0.2 |
| | Methylparaben | 0.1 | 0.1 | 0.1 |
| B | Propylparaben | 0.05 | 0.05 | 0.05 |
| | Xanthane gum | 0.1 | 0.1 | 0.1 |
| | BG | 4.8 | 4.8 | 4.8 |
| | Glycerin | 4.8 | 4.8 | 4.8 |
| | Sodium citrate | 0.4 | 0.4 | 0.4 |
| | Purified water | 60.95 | 60.95 | 61.95 |

(Units in the table are % by mass when the total of A and B is 100)

### (Production Method)

Component A was heated to 85°C, mixed, and then while being stirred with a homomixer, Component B which was heated to 85°C and mixed was gradually added thereto. The mixture was allowed to cool to 60°C, cooled after that, and stirred until reaching 30°C.

### [Comparative Example 1] Cream of Related Art

As a cream of the related art, hydroxycitric acid derivative was replaced with purified water in Formulation 3 of Formulation Example 1.

### [Example 2] Confirmation of Effect on Mitochondrial function Improvement

The effect on mitochondrial function improvement in human skin fibroblasts NB1-RGB cells was measured under the following conditions using a mitochondrial membrane potential as an index.

By performing an operation for 4 days in which confluent NB1-RGB cells were cultured for one hour in a DMEM medium containing 10% fetal bovine serum and 600 µM hydrogen peroxide solution, cells induced of artificial aging by oxidation were prepared as aged cells. These aged cells and normal, unprocessed cells were prepared at a seeding density of 4,000 cells/cm² and cultured for 24 hours in the DMEM medium containing 10% fetal bovine serum. As a specimen, the hydroxycitric acid derivative according to the present invention was dissolved in 50% ethanol to the final concentration of 0 µM (for a control) and 10 µM and added to the DMEM medium containing 10% fetal bovine serum, and culturing was performed for 48 hours. The higher a mitochondrial membrane potential was, the more a fluorescent substance, 5,5',6,6'-tetrachloro-1,1',3,3'-tetraethylbenzimidazoilcarbocyanine iodide (JC-1), permeated the mitochondria, and fluorescent red light was expressed for living cells. The lower a mitochondrial membrane potential was, the less the fluorescent substance permeated the mitochondria, and fluorescent green light was expressed for cells that had died due to apoptosis. JC-1 was added to the medium at a concentration of 10%, cultured for 15 minutes, and it was centrifuged at 400g for 5 minutes after that using JC-1 Mitochondrial Membrane Potential Assay Kit provided by Cayman Chemical. In addition, after changing a medium to assay solution supplied in Assay Kit, it was centrifuged at 400g for 5 minutes, and this process was performed twice. After changing a medium to new assay solution supplied in Assay Kit, a fluorescence intensity of the living cells was measured at an excitation wavelength of 520 to 570 nm and a fluorescence wavelength of 570 to 610 nm, and the dead cells at an excitation wavelength of 485 nm and a fluorescence wavelength of 535 nm.

The test results are shown in Table 2 and Table 3. In order to obtain a ratio of dead cells in which apoptosis had been caused, only the green fluorescence intensity of a cell group to be measured was measured, and relative values, with a measured value of the normal cells as 1, are shown in Table 2.

By referring to the results shown in Table 2, from the fact that fluorescence intensities (relative values) of the dead cells in which apoptosis had been caused were lowered by adding the hydroxycitric acid derivative according to the present invention of Table 2, it was confirmed that an action of suppressing apoptosis increased in fibroblasts in which artificial aging was induced by oxidation stress using hydrogen peroxide.
[Table 2]

**(Table 2) Measurement Results of Cells in which Apoptosis Had been Caused**

| Cell | Specimen | Relative fluorescence intensity |
|---|---|---|
| Normal cell | 50% ethanol | 1.00 |
| Aged cell | 50% ethanol | 1.39 |
| | Hydroxycitric acid-2-palmitate | 1.04 |
| | Hydroxycitric acid-2-myristate | 1.13 |

In order to evaluate the state of mitochondrial membrane potential, red fluorescence intensity and green fluorescence intensity of a cell group to be measured were measured, and values of red fluorescence intensity divided by green fluorescence intensity were calculated, and their relative values, with a measured value of the normal cells as 1, are shown in Table 3.

Here, recovering effect on a mitochondrial membrane potential, which becomes lower with aging, were investigated. It can be said that the higher the difference in mitochondrial membrane potential is, the greater the amount of the energy generation of the mitochondria becomes, and cells are in a healthy state, and therefore these values were set as "effect on mitochondrial function improvement".

By referring to the results shown in Table 3, from the fact that mitochondrial function in the aged cells was enhanced by adding the hydroxycitric acid derivative according to the present invention of Table 3, the effect on mitochondrial function improvement was confirmed.
[Table 3]

**(Table 3) Effect on Mitochondrial Function Improvement**

| Cell | Specimen | Relative fluorescence intensity |
|---|---|---|
| Normal cell | 50% ethanol | 1.00 |
| Aged cell | 50% ethanol | 0.52 |
| | Hydroxycitric acid-2-palmitate | 0.72 |
| | Hydroxycitric acid-2-myristate | 0.66 |

### [Example 3] Confirmation of Effect on Promoting Elastin Production

It is known that elastin is a fiber-like protein, which has a main function of crosslinking collagen, is present in the dermis of the skin, blood vessels, ligaments, and the like, and provides elasticity to the skin. The effect of a hydroxycitric acid derivative on elastin production was examined by the following method.

The aged cells and normal, unprocessed cells which were produced in Example 1 were prepared at a seeding density of 4,000 cells/cm² and cultured for 24 hours in the DMEM medium containing 10% fetal bovine serum. The hydroxycitric acid derivatives according to the present invention which were dissolved in 50% ethanol to the final concentration of 0 µM (for a control), 0.01 µM, and 0.1 µM were added to the DMEM medium containing 10% fetal bovine serum, and culturing was performed for 48 hours. After that, cells were collected, elastin was extracted using Elastin Assay Kit manufactured by Biocolor Ltd., and an absorbance of the extracted solution was measured at a wavelength of 513 nm.

From the results shown in Table 4, it was confirmed that the hydroxycitric acid derivative according to the present invention of Table 4 acts on promoting the elastin production which becomes lower in the aged cells.
[Table 4]

**(Table 4) Effect on Promoting Elastin Production**

| Cell | Specimen | Relative fluorescence intensity |
|---|---|---|
| Normal cell | 50% ethanol | 1.00 |
| Aged cell | 50% ethanol | 0.53 |
| | 0.01 µM Hydroxycitric acid-2-palmitate | 1.11 |
| | 0.1 µM Hydroxycitric acid-2-palmitate | 1.06 |
| | 0.01 µM Hydroxycitric acid-2-myristate | 0.93 |
| | 0.1 µM Hydroxycitric acid-2-myristate | 0.94 |

### [Example 4] Use Test

Using Formulation A and Formulation B, and Formulation C of Comparative Example 1 in the creams of Formulation Example 1, specimens were applied on the skin of each of 20 women (35 to 55 years old) twice a day (in the morning and the evening) for 2 months consecutively. A case where the difference of the evaluation points of the test sites between before use of the creams and after 2 months of consecutive application was 2 points or greater was defined as "effective", a case where the difference was 1 point was defined as "slightly effective", and a case where the difference was 0 points was defined as "ineffective", and the number of subjects who had "effective" or "slightly effective" results was counted as "improvement effect". The effect regarding each item ofmoisture, smoothness, and elasticity of the skin, which are each regarded as a typical functional index for evaluating the effect on anti-aging, was evaluated by the number of subjects who answered to each item of "moisture of the skin", "smoothness of the skin", and "elasticity of the skin" as shown in Table 5.
[Table 5]

**(Table 5) Evaluation criteria**

| Evaluation point | Evaluation |
|---|---|
| 1 | Strongly felt moisture, smoothness, and elasticity of the skin |
| 2 | Felt moisture, smoothness, and elasticity of the skin |
| 3 | Felt a little bit of moisture, smoothness, and elasticity of the skin |
| 4 | Did not feel moisture, smoothness, and elasticity of the skin |

The test results are shown in Table 6. The composition which included the agent for improving mitochondrial function of the present invention shown in Table 1 exhibited excellent effect on anti-aging of the skin. In addition, no one had skin problem during the test, which indicates that there is no safety issue.
[Table 6]

**(Table 6) Result of Skin Use Test**

| | Improvement effect | | |
|---|---|---|---|
| | Moisture of skin | Smoothness of skin | Elasticity of skin |
| Formulation Example 1 | 12 | 15 | 17 |
| Formulation Example 2 | 10 | 12 | 12 |
| Formulation Example 3 | 8 | 5 | 3 |

Each configuration and combinations thereof in each embodiment are merely examples, and additions, omissions, changes, and other modifications of the configurations are possible without departing from the scope of the present invention. The present invention is not limited by the embodiment and only limited by Claims.

### Industrial Applicability

It is possible to provide an agent for improving mitochondrial function capable of improving mitochondrial function, and an anti-aging composition exhibiting effect on preventing skin aging and improving aging symptoms.

## Claims

1. An agent for improving mitochondrial function, comprising:
a hydroxycitric acid derivative represented by the following Formula (I) or a salt thereof, as an active ingredient: 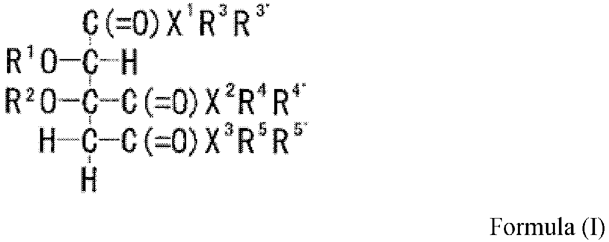 (in Formula (I), R¹ and R² each independently represent a hydrogen atom or any one group selected from the group represented by the following Formula (Ia) (here, R¹ and R² do not represent hydrogen atoms at the same time), X¹ to X³ each independently represent a nitrogen or an oxygen atom, and R³, R⁴, R⁵, R^{3'}, R^{4'}, and R^{5'} each independently represent a hydrogen atom, or a chained hydrocarbon group having 1 to 30 carbon atoms which may have a branch or an unsaturated bond (here, in a case where X¹, X², and X³ each independently represent an oxygen atom, corresponding R^{3'}, R^{4'}, or R^{5'} does not exist), 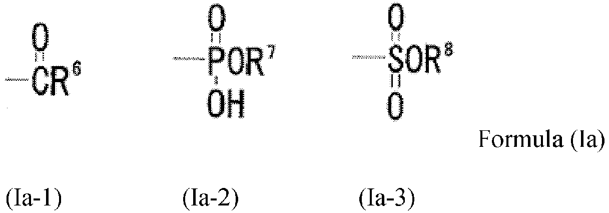 (in Formula (Ia), R⁶ to R⁸ each independently represent a hydrogen atom, an aryl group, or a chained hydrocarbon group having 1 to 30 carbon atoms which may have a branch, an unsaturated bond, or a substituent).

2. The agent for improving mitochondrial function according to Claim 1, wherein in Formula (Ia), R⁶ represents a chained hydrocarbon group having 7 to 23 carbon atoms which may have a branch, an unsaturated bond, or a substituent, and R⁷ and R⁸ each independently represent a hydrogen atom, or a chained hydrocarbon group having 8 to 24 carbon atoms which may have a branch, an unsaturated bond, or a substituent.

3. The agent for improving mitochondrial function according to Claim 1, wherein in Formula (I), X¹ to X³ all represent oxygen atoms, and R³ to R⁵ each independently represent a hydrogen atom, or a chained hydrocarbon group having 1 to 30 carbon atoms which may have a branch or an unsaturated bond, and in Formula (Ia), R⁶ represents a chained hydrocarbon group having 7 to 23 carbon atoms which may have a branch, an unsaturated bond, or a substituent, and R⁷ and R⁸ each independently represent a hydrogen atom, or a chained hydrocarbon group having 8 to 24 carbon atoms which may have a branch, an unsaturated bond, or a substituent.

4. The agent for improving mitochondrial function according to Claim 1, wherein in Formula (I), R² represents a hydrogen atom, X¹ to X³ all represent oxygen atoms, and R³ to R⁵ all represent hydrogen atoms, and in Formula (Ia), R⁶ represents a chained hydrocarbon group having 13 to 21 carbon atoms which may have a branch, an unsaturated bond, or a substituent.

5. The agent for improving mitochondrial function according to Claim 1, wherein in Formula (I), R² represents a hydrogen atom, X¹ to X³ all represent oxygen atoms, and R³ to R⁵ each independently represent a hydrogen atom, or a chained hydrocarbon group having 1 to 30 carbon atoms which may have a branch or an unsaturated bond (here, R³ to R⁵ do not represent hydrogen atoms at the same time), and in Formula (Ia), R⁶ represents a chained hydrocarbon group having 13 to 21 carbon atoms which may have a branch, an unsaturated bond, or a substituent.

6. The agent for improving mitochondrial function according to Claim 1, wherein Formula (I) represents a hydroxycitric acid derivative represented by the following Formula (IIa) or a salt thereof: 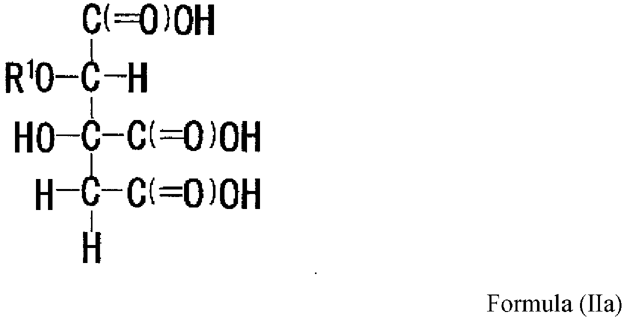 (in Formula (IIa), R¹ represents a group represented by the following Formula (Ia-1)), 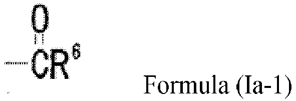 (in Formula (Ia-1), R⁶ represents a chained hydrocarbon group having 7 to 23 carbon atoms which may have a branch, an unsaturated bond, or a substituent).

7. A method for producing the agent for improving mitochondrial function according to Claim 1, the method comprising:
producing the hydroxycitric acid derivative or a salt thereof by reacting hydroxycitric acid and/or an alkali metal salt thereof and/or an alkaline earth metal salt thereof with a carboxylic acid derivative, a phosphoric acid derivative or a sulfonic acid derivative in a solvent.

8. A method for producing the agent for improving mitochondrial function according to Claim 1, the method comprising:
producing the hydroxycitric acid derivative or a salt thereof by the following steps:
a first step of producing a hydroxycitric acid (tri)ester by reacting hydroxycitric acid and/or an alkali metal salt thereof and/or an alkaline earth metal salt thereof with alcohol in a solvent,
a second step of esterifying a hydroxyl group of a compound obtained in the first step by reacting an aliphatic carboxylic acid derivative and the compound obtained in the first step, and
a third step of cleaving a part or all of ester bond moieties formed in the first step from ester bond moieties of the compound obtained in the second step.

9. An anti-aging composition, comprising:
a hydroxycitric acid derivative represented by the following Formula (I) or a salt thereof, as an active ingredient: 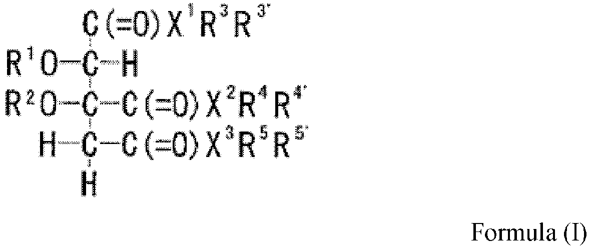 (in Formula (I), R¹ and R² each independently represent a hydrogen atom or any one group selected from the group represented by the following Formula (Ia) (here, R¹ and R² do not represent hydrogen atoms at the same time), X¹ to X³ each independently represent a nitrogen or an oxygen atom, and R³, R⁴, R⁵, R^{3'}, R^{4'}, and R^{5'} each independently represent a hydrogen atom, or a chained hydrocarbon group having 1 to 30 carbon atoms which may have a branch or an unsaturated bond (here, in a case where X¹, X², and X³ each independently represent an oxygen atom, corresponding R^{3'}, R^{4'}, or R^{5'} does not exist), 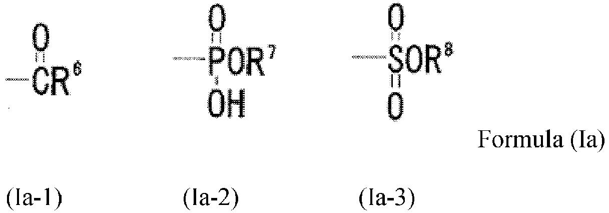 (in Formula (Ia), R⁶ to R⁸ each independently represent a hydrogen atom, an aryl group, or a chained hydrocarbon group having 1 to 30 carbon atoms which may have a branch, an unsaturated bond, or a substituent).
